# EUROPEAN PATENT SPECIFICATION

(11) **EP 2 714 894 B1**
(45) Date of publication and mention of the grant of the patent: **30.10.2019**
(21) Application number: 12726536.1
(22) Date of filing: 25.05.2012
(51) Int. Cl.: C12N 5/071

(54) **BIOARTIFICIAL PROXIMAL TUBULE SYSTEMS AND METHODS OF USE**
BIOARTIFIZIELLE PROXIMALE TUBULUSSYSTEME UND ANWENDUNGSVERFAHREN
SYSTÈMES DE TUBULE PROXIMAL BIOARTIFICIEL ET LEURS PROCÉDÉS D'UTILISATION

(30) Priority: 27.05.2011 US 201161490890 P
(43) Date of publication of application: 09.04.2014
(73) Proprietor: DePuy Synthes Products, Inc., Raynham, MA 02767 (US)
(72) Inventor: KAZANECKI, Christian, Martins Creek, PA 18063 (US); COLTER, David C., Hamilton, NJ 08610 (US); SCHANZ, Johanna, 70184 Stuttgart (DE); HOPPENSACK, Anke, 70732 Stuttgart (DE); HANSMANN, Jan, 70569 Stuttgart (DE); WALLES, Heike, 71063 Sindelfingen (DE)
(74) Representative: Kirsch, Susan Edith
(86) International application number: PCT/US2012/039732
(87) International publication number: WO 2012/166668

(56) References cited:
- WO-A1-01/48153
- US-A- 6 087 157
- US-A1- 2003 165 474
- US-A1- 2008 112 939
- US-A1- 2009 209 019
- NAKAYAMA K H ET AL: "Decellularized Rhesus Monkey Kidney as a Three-Dimensional Scaffold for Renal Tissue Engineering", TISSUE ENGINEERING PART A, vol. 16, no. 7, 1 July 2010 (2010-07-01), pages 2207-2216, XP55034544, ISSN: 1937-3341, DOI: 10.1089/ten.tea.2009.0602

## Description

### FIELD OF THE INVENTION

The invention generally relates to a bioartificial proximal tubule device comprising a biological scaffold and one or more progenitor cells (such as a *e.g.* mammalian kidney-derived cells) that are differentiated into a renal proximal tubule cell monolayer on the scaffold. The invention further relates to the methods of preparing and culturing the device in a bioreactor. Also provided are methods of use of the device for *in vitro* nephrotoxicity or pharmaceutical compound screening.

### BACKGROUND OF THE INVENTION

Chronic kidney disease (CKD) and end-stage renal disease (ESRD) are defined by a decline in renal function, primarily the glomerular filtration rate. This results in an inability of the kidney to excrete toxic metabolic wastes produced by the body. In the United States, CKD is becoming increasingly common and is associated with poor health outcomes and high medical costs. The National Kidney Foundation estimates that 20 million Americans have CKD, and at least 20 million additional people are at risk for developing CKD. ESRD affects over 500,000 patients, with an at-risk population having CKD reaching 1.5 million patients. The total costs for CKD and ESRD account for almost 30% of the total Medicare costs, however these patients make up only 8.1% of the total Medicare population (2008 US Renal Data Service, 2008 Annual Report). The incidence of ESRD has increased over 50% in the past 10 years and the number of patients with or at-risk for CKD is steadily increasing. Accordingly, there is a great need for new therapeutic options to enable repair of damaged kidneys, as well as for *in vitro* systems that can determine the nephrotoxicity of compounds of interest.

Many xenobiotics, or molecules derived from their degradation, are cleared from the blood by active transport into the filtrate destined for the bladder by renal proximal tubule cells of the kidney. As a consequence of carrying out this important function, renal proximal tubule cells are often damaged by the toxic effect of these compounds. Thus, nephrotoxicity testing of potential therapeutic compounds in an *in vitro* system that could potentially replace animal testing has gained significant interest.

Current cell culture based models to test renal proximal tubule epithelial monolayer formation and function utilize primary cells or have been primarily developed using established cell lines from various sources, such as *e.g.* MDCK (Madin-Darby Canine Kidney), LLC-PK1 (Lewis lung cancer porcine kidney 1), or HK-2 cells, which are a human kidney cell line immortalized by transduction with human papilloma virus 16 E6/E7 genes. Assay systems using these cells typically make use of porous filters (such as *e.g*. TRANSWELL® filters), which allow fluid exposure on both the apical and basolateral side of the cells, promoting epithelial differentiation.

However, the use of these cell lines has several disadvantages. Many of these cell lines are transformed or derived from a tumor, potentially altering their growth, differentiation, and ultimately functional characteristics. Furthermore, many of these cell lines are not human-derived. Therefore, there can be species-specific differences in function and in the responses of these cells to various compounds. The use of primary cells is cumbersome as the cells are typically freshly isolated and minimally expanded prior to being used for experiments. The isolation process can be laborious with contamination of unwanted cell populations. In addition, there can be significant variability of the donor source material.

Another common issue is the limited duration in which the primary cells will form an intact monolayer; this limits the cells utility for *in vitro* studies. The primary cells frequently overgrow, pull off the surface and form 3-dimensional aggregates, requiring additional factors to be added, such as MEK inhibitors to maintain contact inhibition (as disclosed in *e.g.* U.S. Pub. App. 2009/0209019). Porous filters (such as *e.g.* TRANSWELL® filters), while somewhat effective, are made from synthetic materials and, therefore, do not accurately represent the underlying matrix that renal tubule cells are typically exposed to *in vivo.*

Others have described alternative methods for screening that rely on the formation of 3-dimensional tubule structures taking advantage of the abovementioned overgrowth of primary cells on solid surfaces (*see* WO 2010/064995 A1), within 3D gels such as *e.g*. MATRIGEL™, or the culture of isolated renal tubules from animals. The latter is problematic due to the laborious isolation technique and species differences that need to be considered. Another disadvantage of these alternatives is their ability to only assess transport of xenobiotic compounds, which are applied to the culture media, into the lumen of the renal tubules. The effect of such compounds on the normal functions of the tubules, such as *e.g.* glucose reabsorption or albumin uptake, cannot be assessed since there is no reliable way to introduce labeled test substances into the lumen or take samples of the luminal fluid of the tubules for assay. In addition, the effects of changes in flow and physiological dynamic conditions that may occur under various *in vivo* scenarios cannot be assessed.

Therefore, there is a need in the field to develop a bioassay that better reflects the normal physiology of renal proximal tubule epithelium. This will ultimately enable the development of new and more effective therapies for renal disease.

### SUMMARY OF THE INVENTION

This application encompasses bioartificial proximal tubule devices having a decellularized biological matrix scaffold on which a monolayer of renal proximal tubule cells is formed from precursor cells (such as *e.g.* mammalian (*e.g.* human) kidney-derived cells).

The present invention describes a bioartificial proximal tubule device, constructed by preparing a decellularized biological matrix, seeding the biological matrix with mammalian kidney-derived cells and optionally mammalian endothelial cells. The device may then be cultured statically or matured using bioreactor culture to allow differentiation of the kidney cells into functioning proximal tubule cells. The resulting device is capable of carrying out proximal tubule functions, for example, the transport of molecules from either side of the biological membrane to the other. The present invention also describes various methods of making and maturing the bioartificial proximal tubule devices. The present invention also describes methods of use of the bioartificial proximal tubule devices for *in vitro* studies of tubule cell transport, toxicity effects of various compounds or pharmaceutical compound screening.

In one embodiment, the bioartificial proximal tubule device comprises a decellularized biological matrix scaffold seeded with a one or more cells differentiable into renal cells (*e.g.* a precursor cell that can differentiate into renal cells) under conditions sufficient to allow the differentiation of these cells into renal proximal tubule cells whereby the differentiated cells form an epithelial monolayer on the scaffold. The bioartificial proximal tubule device may optionally further comprise vascular endothelial cells.

In another embodiment, the bioartificial proximal tubule device comprises a decellularized biological scaffold having at least two surfaces wherein at least one surface is seeded with one or more cells differentiable into renal cells (*e.g.* a precursor cell that can differentiate into a renal cell) under conditions sufficient to allow differentiation of the cells into renal proximal tubule epithelial cells, whereby the cells form a cell monolayer on the surface of the scaffold.

In an alternate embodiment, the bioartificial proximal device comprises an decellularized biological scaffold derived from mammalian tissue having one or more surfaces and a renal proximal tubule epithelial monolayer on a surface of the scaffold, wherein the epithelial monolayer is formed by seeding the surface with one or more mammalian kidney-derived cells under conditions sufficient to allow differentiation of the kidney-derived cells into renal proximal tubule cells and formation of the monolayer. The seeding of the surface may be carried out in a bioreactor. Such a bioreactor may have an upper body element, a lower body element with an area for cell growth, and one or more connectors.

The one or more cells differentiable into renal cells (*e.g*. precursor cell) may be primary renal tubule epithelial cells, inducible pluripotent stem cells or progenitor cells differentiated into renal cells or renal progenitor cells, stem cells isolated from the kidney or progenitor cells isolated from the kidney, and mixtures thereof. In one embodiment, the one or more cells differentiable into renal cells are kidney-derived cells from a mammal such as *e.g.* a human. These kidney-derived cells may be obtained from the kidney cortex, kidney medulla, kidney subcapsular region and mixtures thereof.

In yet another embodiment, the bioartificial proximal tubule device comprises a decellularized biological scaffold having at least two surfaces wherein at least one surface is seeded with one or more mammalian kidney-derived cells under conditions sufficient to allow differentiation of the kidney-derived cells into renal proximal tubule cells, wherein the cells form an epithelial monolayer on the surface of the scaffold. The mammal may be a human and the cells may be obtained from the kidney cortex, kidney medulla or kidney subcapsular region.

The decellularized biological matrix scaffold is derived from mammalian tissue. The scaffold is derived from mammalian tissue such as *e.g.* porcine tissue. For example, the scaffold may be derived from the stomach, duodenum, jejunum, ileum or colon of a mammal. In one embodiment of the invention, the scaffold is derived from small intestine submucosa. In another embodiment, the decellularized biological matrix may be derived from mucosal or submucosal tissue.

In one embodiment of the invention, the kidney-derived cells are capable of self-renewal and expansion in culture, positive for the expression of one or more of Oct-4, Pax-2 and Rex-1 and negative for the expression of one or more of Sox2, FGF4, hTert and Wnt-4. In another embodiment, the kidney-derived cells are capable of self-renewal and expansion in culture, positive for the expression of one or more of Oct-4 and Pax-2 and negative for the expression of one or more of Sox2, FGF4, hTert and Wnt-4.

In another embodiment, the kidney-derived cells are capable of self-renewal and expansion in culture, positive for expression of at least one of Eyal, Pax-2, WT1, FoxD1, BMP7, BMP2, GDF5, EpoR or Rex-1, and negative for expression of at least one of Sox2, FGF4, hTert or Wnt-4. In certain embodiments, the kidney-derived cells may also be positive for at least one of cell-surface markers HLA I, CD24, CD29, CD44, CD49c, CD73, CD90, CD166, or SSEA-4, and negative for at least one of cell-surface markers HLA II, CD31, CD34, CD45, CD56, CD80, CD86, CD104, CD105, CD117, CD133, CD138, CD141, or E-cadherin.

In some embodiments, a second surface of the scaffold may be seeded with mammalian vascular endothelial cells. For example, the vascular endothelial cells may be endothelial cells lines, endothelial progenitor cells, primary endothelial cells, microvascular endothelial cells and mixtures thereof.

An alternate embodiment of the invention is a bioartificial proximal tubule device comprising a decellularized biological matrix scaffold seeded with one or more precursor cells (*i.e.* precursor cells which can differentiate into renal cells) under conditions sufficient to allow the differentiation of the precursor cell into renal proximal tubule epithelial cells, wherein the differentiated cells form an epithelial monolayer on the scaffold. The decellularized biological matrix scaffold may be derived from mammalian tissue (*e.g.* porcine tissue) such as *e.g.* mucosal or submucosal tissue. In one embodiment, decellularized biological matrix scaffold is derived from a mammalian alimentary canal. In another embodiment, the decellularized biological matrix scaffold is derived (*e.g*. obtained) from the stomach, duodenum, jejunum, ileum or colon of a mammal. The one or more precursor cells is selected from the group consisting of primary renal tubule epithelial cells, inducible pluripotent stem cells differentiated into renal cells or renal progenitor cells, progenitor cells differentiated into renal cells or renal progenitor cells, stem cells isolated from the kidney or progenitor cells isolated from the kidney, and mixtures thereof. In one embodiment, the progenitor cells are human kidney-derived cells. In one embodiment, the human kidney-derived cells are capable of self-renewal and expansion in culture and are positive for expression of at least one of Oct-4, Rex-1, Pax-2, Cadherin-11, FoxD1, WT1, Eya1, HNF3B, CXC-R4, Sox-17, EpoR, BMP2, BMP7, or GDF5; and negative for the expression of at least one of Sox2, FGF4, hTert, Wnt-4, SIX2, E-cadherin or GATA-4. Optionally, these cells are also positive for at least one of cell-surface markers HLA-I, CD24, CD29, CD44, CD49c, CD73, CD90, CD166, or SSEA-4; and negative for at least one of cell-surface markers HLA II, CD31, CD34, CD45, CD56, CD80, CD86, CD104, CD105, CD117, CD133, CD138, and CD141. In addition, the cells optionally further secrete at least one of trophic factors FGF2, HGF, TGFα, TIMP-1, TIMP-2, MMP-2 or VEGF; and do not secrete at least one of trophic factors PDGF-bb or IL12p70.

Yet another alternate embodiment of the invention is a bioartificial proximal tubule device comprising a decellularized biological scaffold having at least two surfaces wherein at least one surface is seeded with one or more precursor cells (*i.e.* precursor cells which can differentiate into renal cells) under conditions sufficient to allow differentiation of the cells into renal proximal tubule epithelial cells, wherein the cells form an epithelial monolayer on the surface of the scaffold. The decellularized biological matrix scaffold is derived from mammalian tissue (*e.g*. porcine tissue). In one embodiment the decellularized biological matrix scaffold is derived from mucosal or submucosal tissue. Alternatively, the decellularized biological matrix scaffold is derived from a mammalian alimentary canal. In another embodiment, the decellularized biological matrix scaffold is derived (*e.g*. obtained) from the stomach, duodenum, jejunum, ileum or colon of a mammal. The one or more precursor cells is selected from the group consisting of primary renal tubule epithelial cells, inducible pluripotent stem cells differentiated into renal cells or renal progenitor cells, progenitor cells differentiated into renal cells or renal progenitor cells, stem cells isolated from the kidney or progenitor cells isolated from the kidney, and mixtures thereof. In one embodiment, the progenitor cells are human kidney-derived cells. In one embodiment, the human kidney-derived cells are capable of self-renewal and expansion in culture and are positive for expression of at least one of Oct-4, Rex-1, Pax-2, Cadherin-11, FoxD1, WT1, Eyal, HNF3B, CXC-R4, Sox-17, EpoR, BMP2, BMP7, or GDF5; and negative for the expression of at least one of Sox2, FGF4, hTert, Wnt-4, SIX2, E-cadherin or GATA-4. Optionally, these cells are also positive for at least one of cell-surface markers HLA-I, CD24, CD29, CD44, CD49c, CD73, CD90, CD166, or SSEA-4; and negative for at least one of cell-surface markers HLA II, CD31, CD34, CD45, CD56, CD80, CD86, CD104, CD105, CD117, CD133, CD138, and CD141. In addition, the cells optionally further secrete at least one of trophic factors FGF2, HGF, TGFα, TIMP-1, TIMP-2, MMP-2 or VEGF; and do not secrete at least one of trophic factors PDGF-bb or IL12p70. In another embodiment, the second surface of the scaffold is seeded with mammalian vascular endothelial cells. These vascular endothelial cells may be selected from endothelial cells lines, endothelial progenitor cells, primary endothelial cells or microvascular endothelial cells.

Yet another embodiment of the invention is a method of using the proximal tubule devices. Accordingly, one embodiment is a method of differentiating one or more precursor cells into renal cells comprising seeding a decellularized biological matrix scaffold (*i*.*e.* precursor cells which can differentiate into renal cells) with one or more precursor cells and culturing the cells on the scaffold under conditions sufficient to allow the differentiation of the precursor cell into renal proximal tubule epithelial cells, wherein the differentiated cells form an epithelial monolayer on the scaffold. The decellularized biological matrix scaffold may have two surfaces. In one embodiment, the decellularized biological matrix scaffold is derived from mammalian (*e.g*. porcine) tissue. Accordingly, the decellularized biological matrix scaffold may be derived (*e.g*. obtained) from mucosal or submucosal tissue. In another embodiment, the decellularized biological matrix scaffold is derived from the stomach, duodenum, jejunum, ileum or colon of a mammal. The methods may utilize the one or more precursor cells selected from the group consisting of primary renal tubule epithelial cells, inducible pluripotent stem cells differentiated into renal cells or renal progenitor cells, progenitor cells differentiated into renal cells or renal progenitor cells, stem cells isolated from the kidney or progenitor cells isolated from the kidney, and mixtures thereof. In one embodiment, the progenitor cells utilized in the methods are human kidney-derived cells.

In one embodiment, the human kidney-derived cells used in the methods are capable of self-renewal and expansion in culture and are positive for expression of at least one of Oct-4, Rex-1, Pax-2, Cadherin-11, FoxD1, WT1, Eyal, HNF3B, CXC-R4, Sox-17, EpoR, BMP2, BMP7, or GDF5; and negative for the expression of at least one of Sox2, FGF4, hTert, Wnt-4, SIX2, E-cadherin or GATA-4. In another embodiment, these cells are also positive for at least one of cell-surface markers HLA-I, CD24, CD29, CD44, CD49c, CD73, CD90, CD166, or SSEA-4; and negative for at least one of cell-surface markers HLA II, CD31, CD34, CD45, CD56, CD80, CD86, CD104, CD105, CD117, CD133, CD138, and CD141. In another embodiment, the cells secrete at least one of trophic factors FGF2, HGF, TGFα, TIMP-1, TIMP-2, MMP-2 or VEGF; and do not secrete at least one of trophic factors PDGF-bb or IL12p70.

Another embodiment of the invention is a method of differentiating one or more cells differentiable into renal cells into renal proximal tubule epithelial cells under conditions sufficient to allow the differentiation, whereby the differentiated cells form an epithelial monolayer and whereby tension is applied to the cells. The method may further comprise the step of seeding the cells on a decellularized scaffold prior to differentiating the cells. When the method comprises this step, the method can be used to produce a bioartificial device of the invention. The decellularized biological matrix scaffold may be derived from mammalian (such as *e.g.* porcine) tissue such as mucosal or submucosal tissue. In one embodiment, the decellularized biological matrix scaffold is derived from a mammalian alimentary canal. In another embodiment, the decellularized biological matrix scaffold is derived from the stomach, duodenum, jejunum, ileum or colon of a mammal. The one or more cells differentiable into renal cells is selected from the group consisting of primary renal tubule epithelial cells, inducible pluripotent stem cells or progenitor cells differentiated into renal cells or renal progenitor cells, stem cells isolated from the kidney or progenitor cells isolated from the kidney and mixtures thereof. In one embodiment, the one or more cells differentiable into renal cells are mammalian kidney-derived cells (such as *e.g*. human kidney-derived cells). The mammalian kidney-derived cells may be obtained from the kidney cortex, kidney medulla, kidney subcapsular region and mixtures thereof. In one embodiment, the kidney-derived cells are capable of self-renewal and expansion in culture, positive for the expression of one or more of Oct-4, Pax-2, and Rex-1 and negative for the expression of one or more of Sox2, FGF4, hTert and Wnt-4. In an alternate embodiment, the kidney-derived cells are capable of self-renewal and expansion in culture, positive for expression of at least one of Eyal, Pax2, WT1, FoxD1, BMP7, BMP2, GDF5, EpoR or Rex-1, and negative for expression of at least one of Sox2, FGF4, hTert or Wnt-4. In yet another embodiment, the kidney-derived cells are also positive for at least one of cell-surface markers HLA I, CD24, CD29, CD44, CD49c, CD73, CD166, or SSEA-4, and negative for at least one of cell-surface markers HLA II, CD31, CD34, CD45, CD56, CD80, CD86, CD104, CD105, CD117, CD133, CD138, CD141, or E-cadherin.

### BRIEF DESCRIPTION OF THE DRAWINGS

The foregoing summary, as well as the following detailed description of the invention, will be better understood when read in conjunction with the appended figures. For the purpose of illustrating the invention, the figures demonstrate embodiments of the present invention. It should be understood, however, that the invention is not limited to the precise arrangements, examples, and instrumentalities shown.

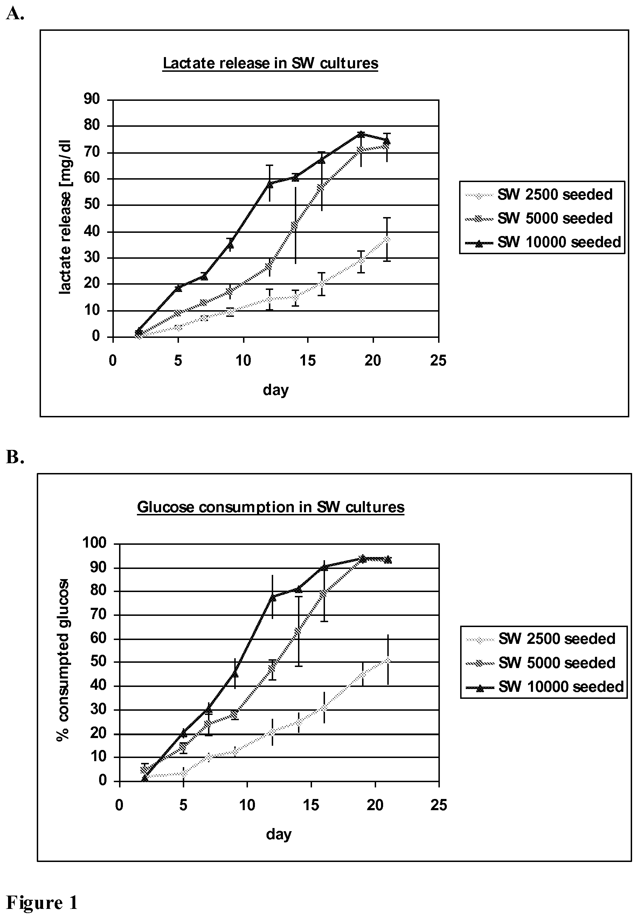

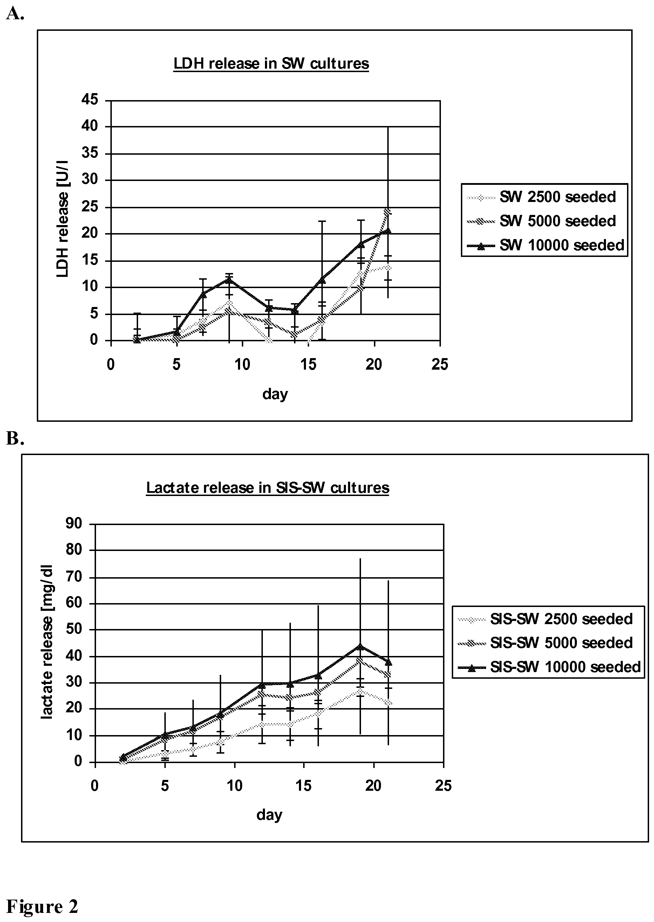

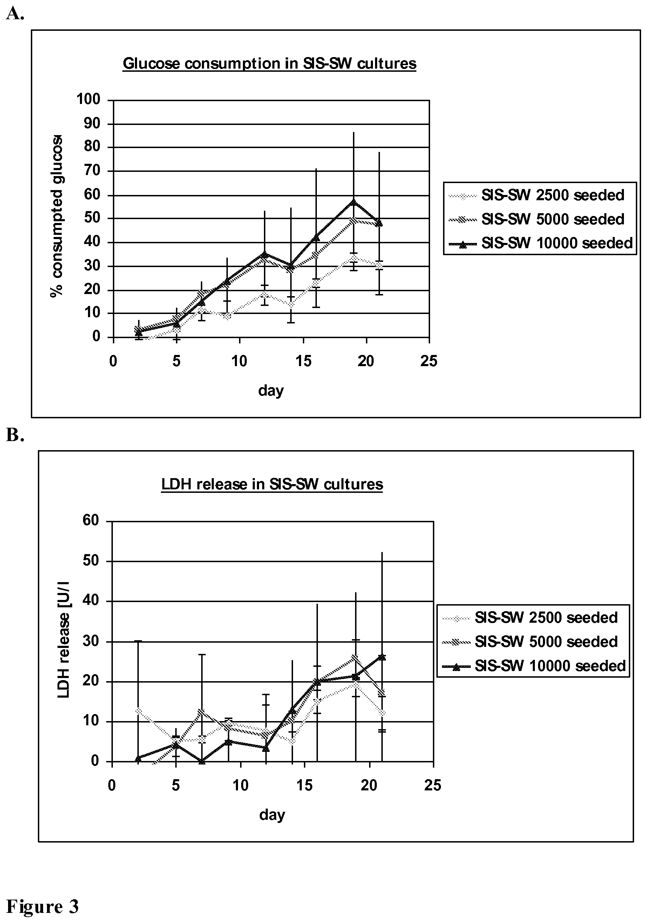

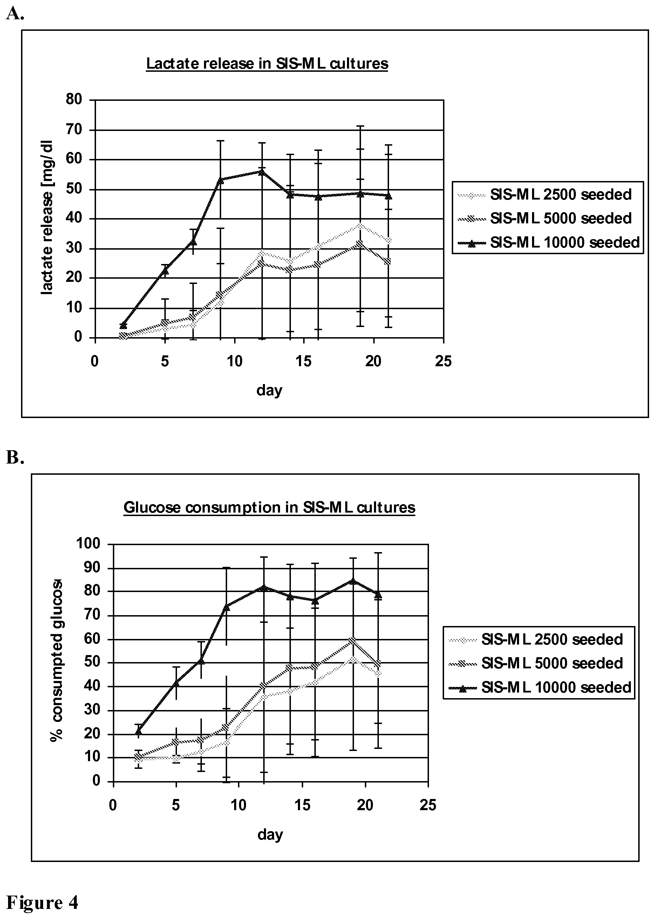

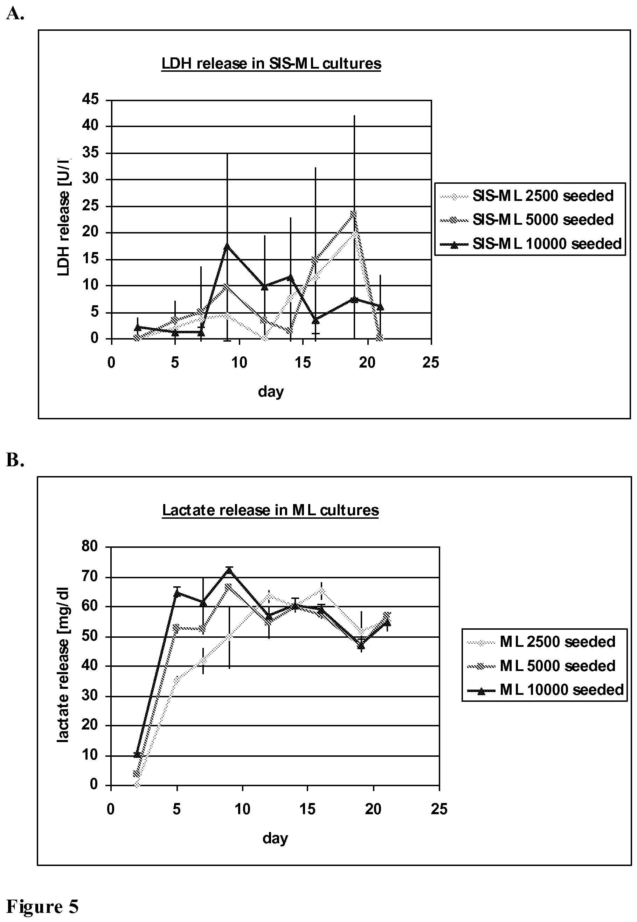

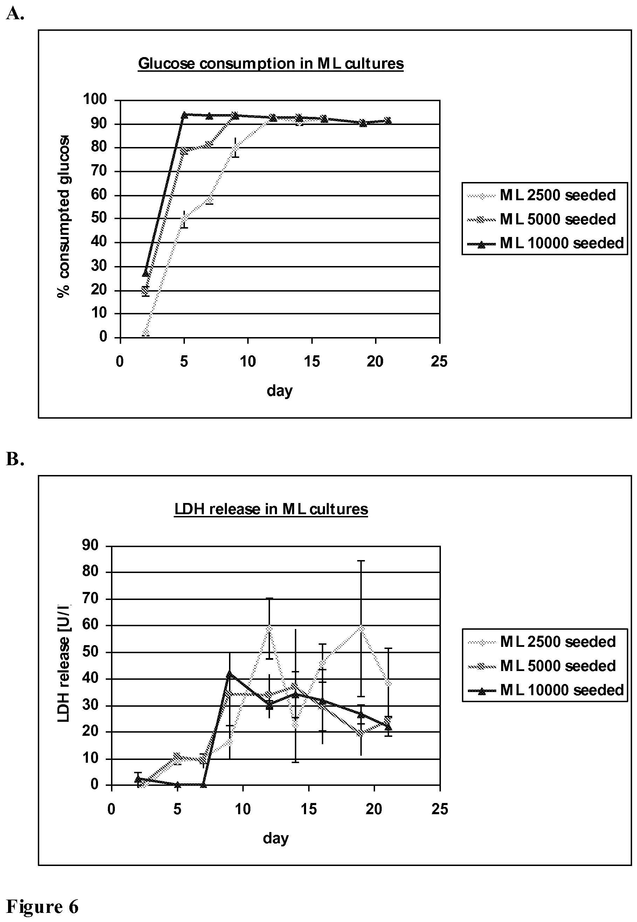

Figures 1 to 6 show the results of the analysis of metabolic parameters for human kidney-derived cells. Figure 1A shows the lactate release in human kidney-derived cell cultures ("SW cultures") as a function of time. Figure 1B shows the glucose consumption in SW cultures as a function of time. Figure 2A shows the LDH release in SW cultures as a function of time. Figure 2B shows the lactate release in cell cultures of human kidney-derived cells seeded on decellularized small intestine submucosa (SIS) ("SIS-SW cultures") as function of time. Figure 3A shows the glucose consumption in SIS-SW cultures as a function of time. Figure 3B shows the LDH release in SIS-SW cultures as a function of time. Figure 4A shows the lactate release in monolayers grown from human kidney-derived cells which were seeded on decellularized small intestine submucosa (SIS) ("SIS-ML cultures") as a function of time. Figure 4B shows the glucose consumption in SIS-ML cultures as a function of time. Figure 5A shows the LDL release in SIS-ML cultures as a function of time. Figure 5B shows the lactate release in ML cultures as a function of time. Figure 6A shows the glucose consumption in ML cultures as a function of time. Figure 6B shows the LDH release in ML cultures as a function of time. In Figures 1 to 6, n = 3.

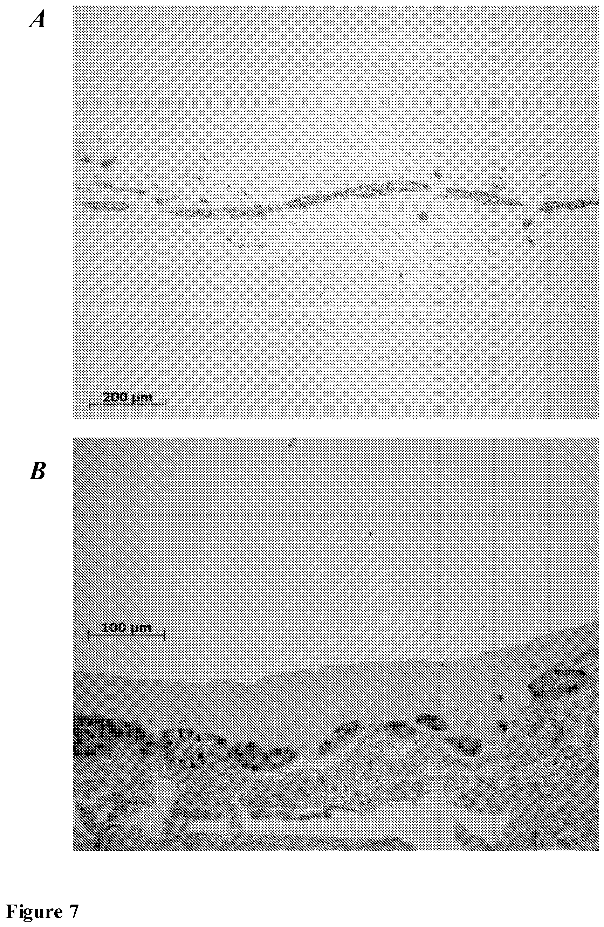

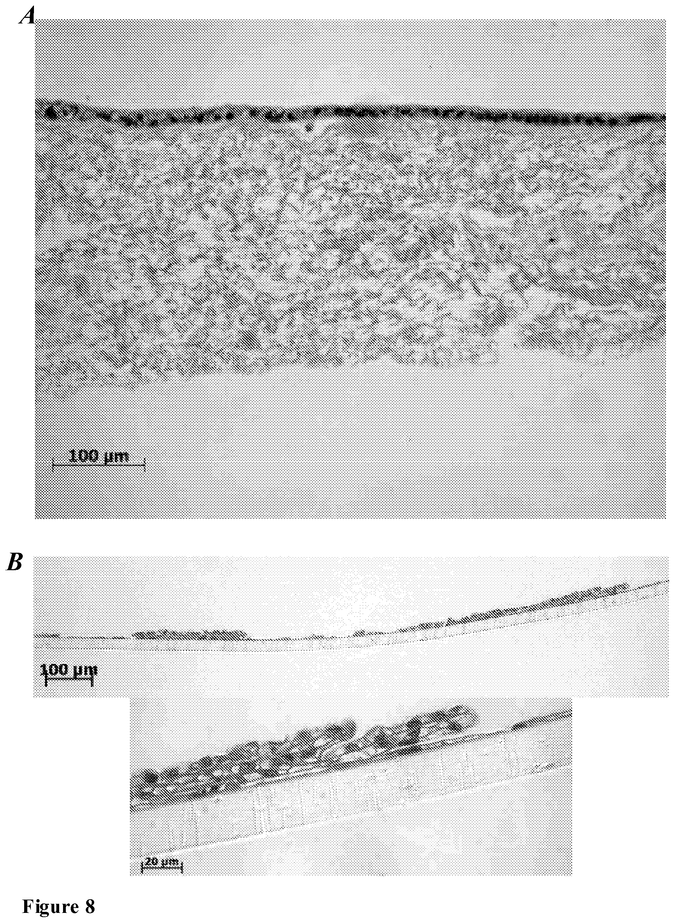

Figure 7 and Figure 8 show histological staining of human kidney-derived cells (hKDCs) on extracellular (*i.e.* decellularized) matrix scaffolds. Human kidney-derived cells were seeded onto three different scaffold configurations at a concentration of 2.5 x 10³ cells/scaffold and cultivated for three weeks. The samples were then fixed and the slices were stained with hematoxylin and eosin (H&E). Figure 7A shows histological staining of the collagen sandwich culture. Figure 7B shows histological staining of the collagen-decellularized small intestine submucosa (SIS) sandwich culture. Figure 8A shows histological staining of the decellularized SIS monolayer culture. Figure 8B shows histological staining of the collagen-coated transwell culture.

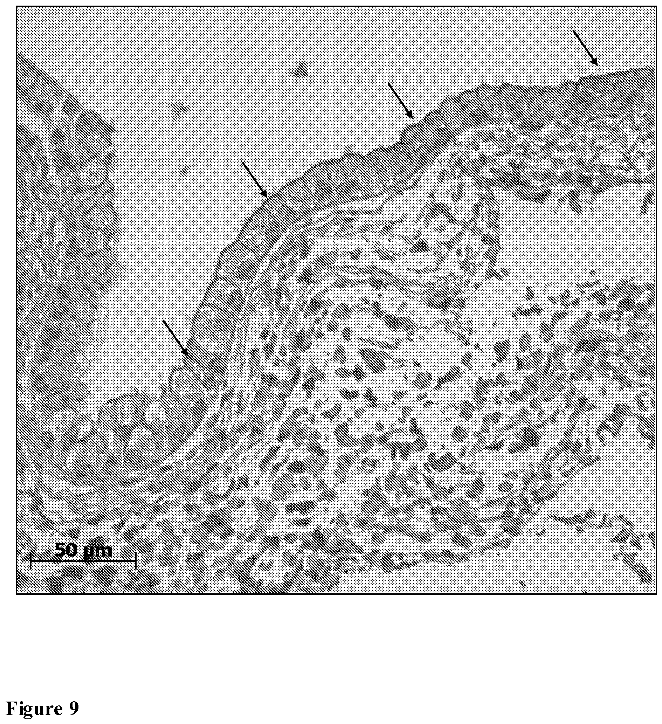

Figure 9 shows immunohistochemical detection of Aquaporin-1 by hKDCs seeded on extracellular (decellularized) matrix scaffolds. Human-derived kidney cells were seeded onto decellularized SIS scaffolds and allowed to attach overnight. The samples were cultured for three weeks, then fixed. Subsequently, 3 µm thick slices were used for immunohistochemistry (IHC) with an anti-human Aquaporin-1 antibody (Abcam, Cambridge). The arrows indicate areas of apical staining of the cells.

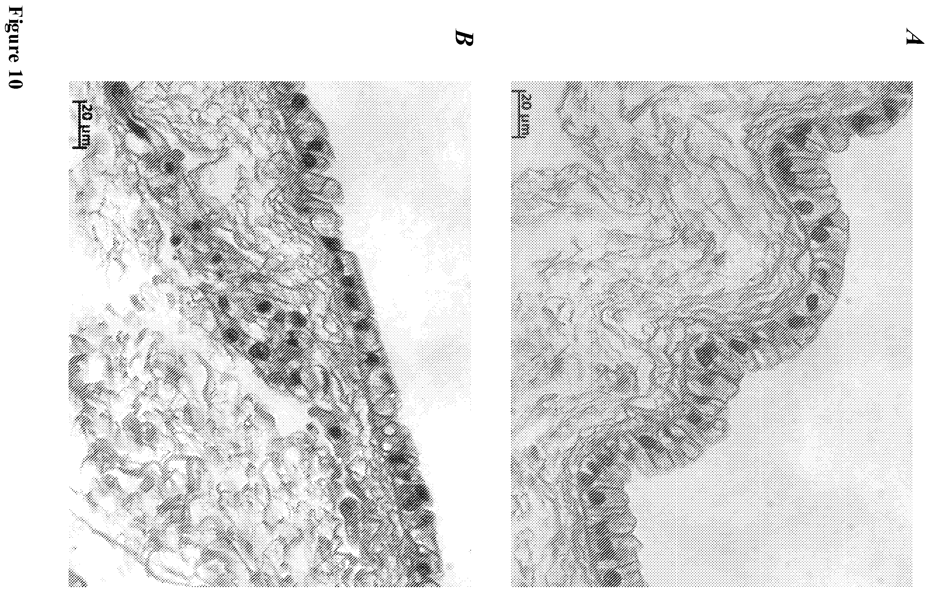

Figure 10 shows the histological staining of hKDCs seeded on decellularized scaffolds. Human kidney-derived cells were seeded onto decellularized SIS scaffolds at two different cell concentrations and cultivated for three weeks, then fixed. Subsequently, 3 µm thick slices were stained with hematoxylin and eosin (H&E). Figure 10A shows the H&E staining of a sample seeded with 1 x 10⁴ cells. Figure 10B shows the H&E staining of a sample seeded with 5 x 10⁴ cells.

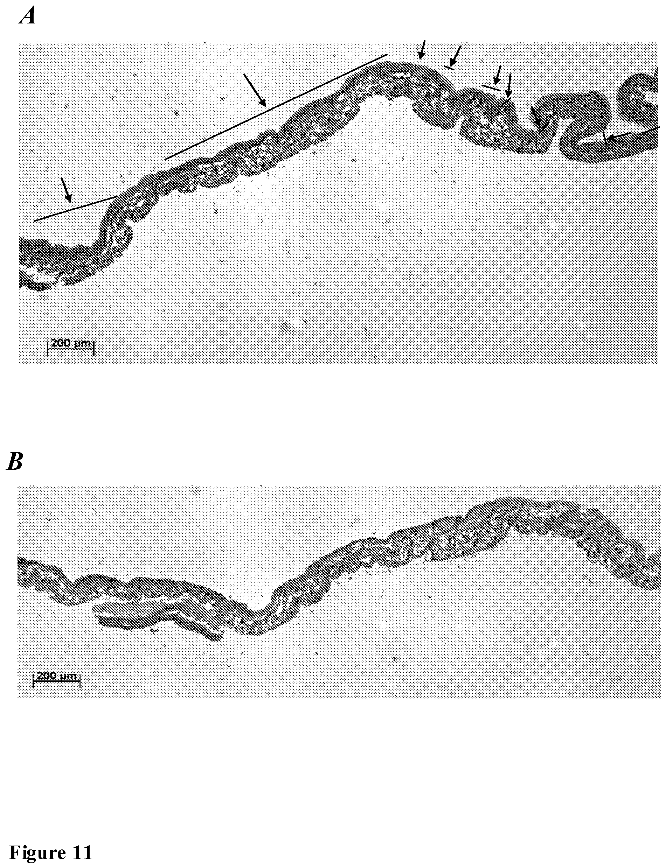

Figure 11 shows the lectin staining of three-week cultures of hKDC seeded on decellularized scaffolds. Human kidney-derived cells were seeded onto decellularized SIS scaffolds and cultivated for three weeks, then fixed. Subsequently, 3 µm thick slices were stained with *Lotus tetragonobulus* lectin (Figure 11A) and *Dolichos biflorus* agglutinin (Figure 11B). Arrows and lines in Figure 11A indicate areas of positive staining.

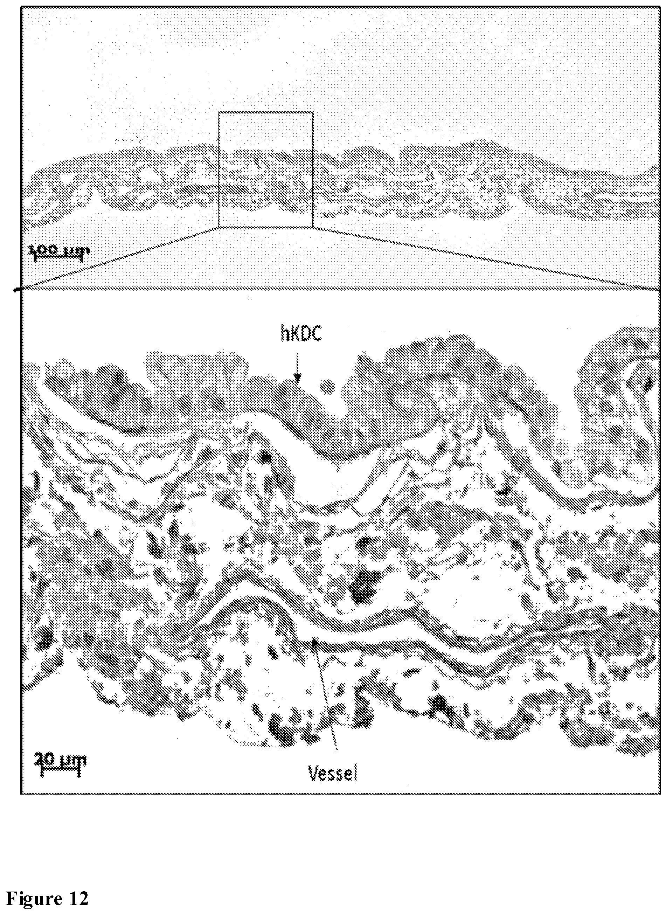

Figure 12 shows the Collagen IV staining of hKDC seeded on decellularized scaffolds. Human kidney-derived cells were seeded onto decellularized SIS scaffolds, cultivated for three weeks, and then fixed. Subsequently, 3 µm thick slices were stained with anti- collagen IV antibody.

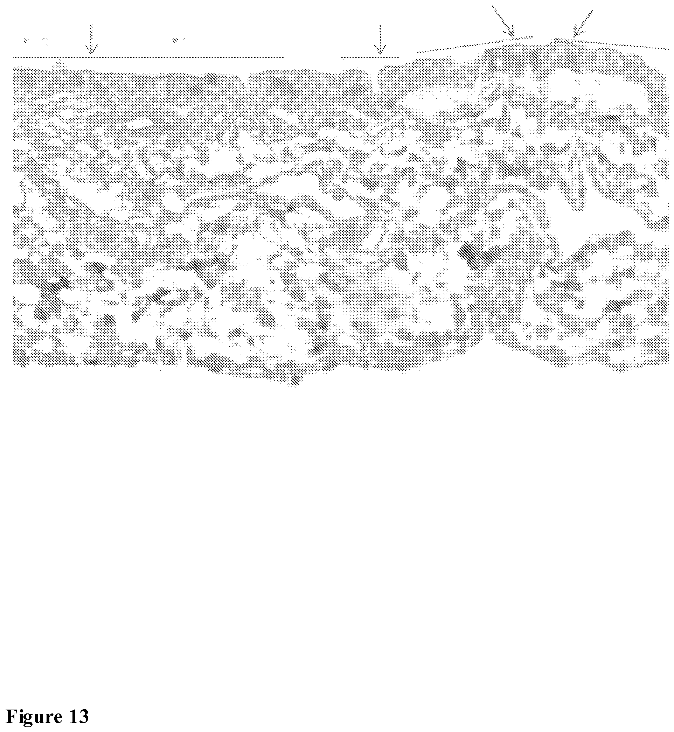

Figure 13 shows the Pgp-1 staining of hKDC seeded on decellularized scaffolds. Human kidney-derived cells were seeded onto decellularized SIS scaffolds and cultivated for three weeks, then fixed. Subsequently, 3 µm thick slices were stained with ananti-human P-glycoprotein-1 antibody.

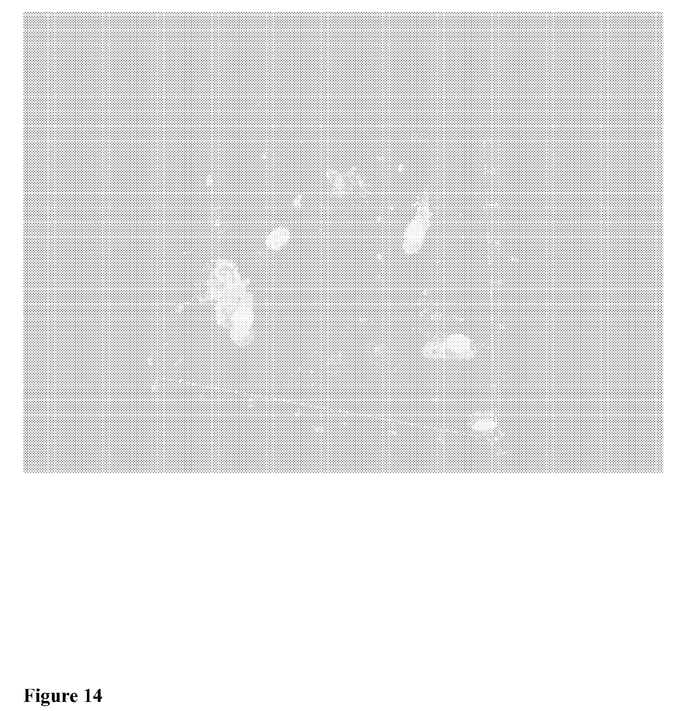

Figure 14 shows the uptake of fluorescently labeled bovine serum albumin (BSA-FITC) by hKDC seeded on decellularized scaffolds. Human kidney-derived cells were seeded onto decellularized SIS scaffolds, cultivated for two weeks, and then cultured in the presence of BSA-FITC for 1 hour. Subsequently, the cells were counterstained with DAPI (diamidino-2-phenylindole) and imaged.

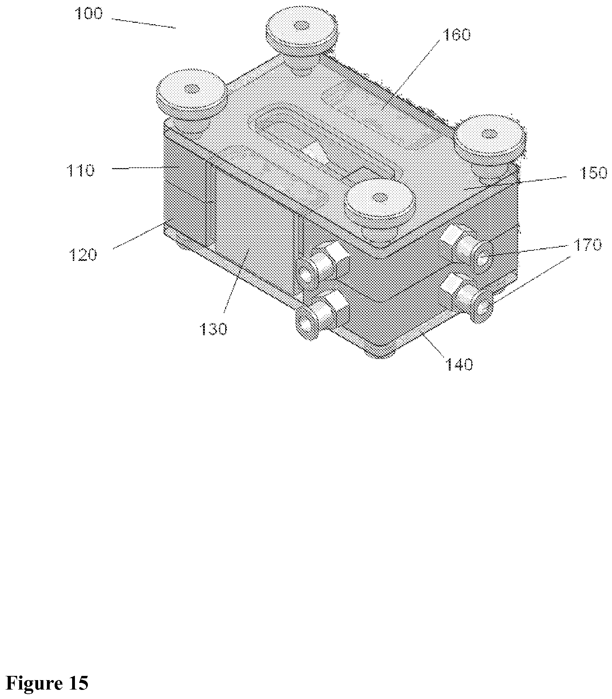

Figure 15 is a schematic of a bioreactor for cell cultivation showing the main reactor components.

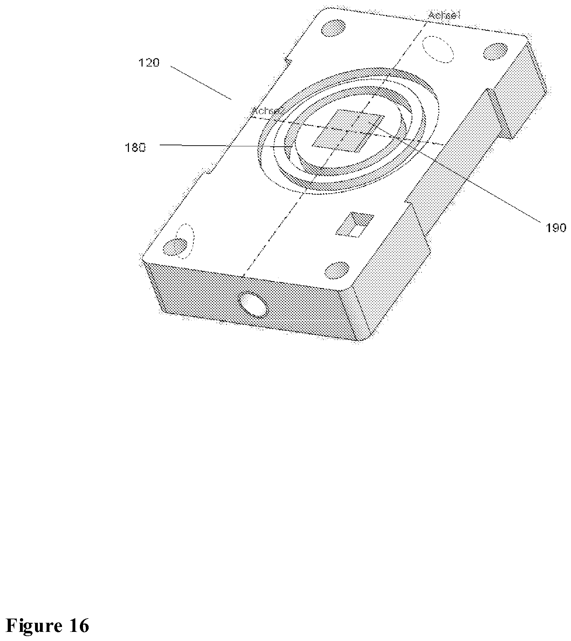

Figure 16 shows a detailed view of the lower body element of the bioreactor.

### DETAILED DESCRIPTION

Various terms relating to the device, methods of using the device and other aspects of the invention are used throughout the specification and the claims. Such terms are to be given their ordinary meaning in the art unless otherwise indicated. Other specifically defined terms are to be construed in a matter consistent with the definition provided herein.

This invention is based on the discovery that precursor cells that can differentiate into renal cells (cells differentiable into renal cells) (such *e.g*. kidney-derived cells), when seeded on a decellularized biological matrix scaffold under conditions that allow differentiation, form a renal proximal tubule epithelial monolayer on the surface of the scaffold.

It is to be understood that this invention is not limited to particular methods, reagents, compounds, compositions or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a cell" includes a combination of two or more cells, and the like.

As used herein, the term "about" when referring to a measurable value such as an amount, a temporal duration, and the like, is meant to encompass variations of ± 20% or ± 10%, more preferably ± 5%, even more preferably ± 1%, and still more preferably ± 0.1% from the specified value, as such variations are appropriate to perform the disclosed methods.

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

"Differentiation" is the process by which an unspecialized ("uncommitted") or less specialized cell acquires the features of a specialized cell, such as a kidney cell, for example. A "differentiated or differentiation-induced cell" is one that has taken on a more specialized ("committed") position within the lineage of a cell. The term "committed," when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. "De-differentiation" refers to the process by which a cell reverts to a less specialized (or committed) position within the lineage of a cell. As used herein, the "lineage" of a cell defines the heredity of the cell, *i.e.,* which cells it came from and what cells it can give rise to. The lineage of a cell places the cell within a hereditary scheme of development and differentiation. A "lineage-specific marker" refers to a characteristic specifically associated with the phenotype of cells of a lineage of interest and can be used to assess the differentiation of an uncommitted cell to the lineage of interest.

In a broad sense, a "progenitor cell" is a cell that has the capacity to create progeny that are more differentiated than itself and yet retains the capacity to replenish the pool of progenitors. By that definition, stem cells themselves are also progenitor cells, as are the more immediate precursors to terminally differentiated cells. When referring to the cells disclosed herein, this broad definition of "progenitor cell" may be used. A differentiated cell can be derived from a multipotent cell which itself is derived from a multipotent cell, and so on. While each of these multipotent cells can be considered stem cells, the range of cell types each can give rise to may vary considerably. Some differentiated cells also have the capacity to give rise to cells of greater developmental potential. Such capacity can be natural or can be induced artificially upon treatment with various factors. "Proliferation" indicates an increase in cell number.

"Kidney progenitor cells" as used herein are mammalian (*e.g*. human) kidney-derived cells that can give rise to cells, such as adipocytes, or osteoblasts or can give rise to one or more types of tissue, for example, renal tissue, in addition to producing daughter cells of equivalent potential. A "kidney or renal progenitor cell" is a multipotent or pluripotent cell that originates substantially from adult or fetal kidney tissue. These cells have been found to possess features characteristic of pluripotent stem cells, including rapid proliferation and the potential for differentiation into other cell lineages. "Multipotent" kidney progenitor cells can give rise to multiple cell lineages, *e.g*., renal cell lineages, adipocyte lineages, or osteoblast lineages. Kidney progenitor cells demonstrate a gene expression profile for early developmental gene markers, kidney developmental gene markers, metanephric mesenchymal gene markers, and genes that promote the survival of metanephric mesenchyme. For example, kidney progenitor cells (*e.g*. human kidney-derived cells) demonstrate a gene expression profile which is positive for expression of genes including, but not limited to, Oct-4, Pax-2 and Rex-1, and negative for expression of genes including, but not limited to, Sox2, FGF4, hTERT and Wnt-4.

Tissue" refers to a group or layer of similarly specialized cells, which together perform certain special functions. "Organ" refers to two or more adjacent layers of tissue, which layers of tissue maintain some form of cell-cell and/or cell-matrix interaction to form a microarchitecture.

"Kidney" refers to one of a pair of organs in the abdomen. Kidneys remove waste from the blood (as urine), produce erythropoietin to stimulate red blood cell production, and play a role in blood pressure regulation. Kidneys function to maintain proper water and electrolyte balance, regulate acid-base concentration, and filter the blood of metabolic wastes, which are then excreted as urine.

"Primary culture" refers to a mixed cell population of cells that permits interaction of many different cell types isolated from a tissue. The word "primary" takes its usual meaning in the art of tissue culture. "Capable of self-renewal and expansion in culture" refers to mammalian kidney-derived cell populations that grow and divide in cell culture and maintain substantially the same phenotype as measured by cell markers and secretion of trophic factors from mother cell to daughter cell. At some point during replication of the mammalian kidney-derived cell population, the phenotype can change to a more specialized or differentiated state of the kidney-derived cell.

Various terms are used to describe cells in culture. "Cell culture" refers generally to cells taken from a living organism and grown under controlled conditions, *e.g.,* "in culture". A "primary cell culture" is a culture of cells, tissues or organs taken directly from organisms and before the first subculture. Cells are "expanded" in culture when they are placed in a growth medium under conditions that facilitate cell growth and/or division, resulting in a larger population of the cells. When cells are expanded in culture, the rate of cell proliferation is sometimes measured by the amount of time needed for the cells to double in number. This is referred to as "doubling time."

A "cell line" is a population of cells formed by one or more subcultivations of a primary cell culture. Each round of subculturing is referred to as a passage. When cells are subcultured, they are referred to as having been "passaged." A specific population of cells, or a cell line, is sometimes referred to or characterized by the number of times it has been passaged. For example, a cultured cell population that has been passaged ten times may be referred to as a "P10" culture. The primary culture, *i.e.,* the first culture following the isolation of cells from tissue, is designated P0. Following the first subculture, the cells are described as a secondary culture (PI or passage 1). After the second subculture, the cells become a tertiary culture (P2 or passage 2), and so on. It will be understood by those of skill in the art that there may be many population doublings during the period of passaging; therefore the number of population doublings of a culture is usually greater than the passage number. The expansion of cells (*i.e.,* the number of population doublings) during the period between passaging depends on many factors, including, but not limited to the seeding density, substrate, medium, and time between passaging.

Generally, a "trophic factor" is defined as a substance that promotes survival, growth, proliferation, maturation, differentiation, and/or maintenance of a cell, or stimulates increased activity of a cell. "Trophic support" is used herein to refer to the ability to promote survival, growth, proliferation, maturation, differentiation, and/or maintenance of a cell, or to stimulate increased activity of a cell. The mammalian kidney-derived cell population of the present invention produces trophic factors, including but not limited to, growth factors, cytokines, and differentiation factors. The trophic factors include, but are not limited to, FGF2, HGF, TGFα, TIMP-1, TIMP-2, VEGF, MMP-2, or a combination thereof.

"Non-immunogenic" refers to cells or a cell population that does not elicit a deleterious immune response in a majority of treated mammalian subjects, that is an immune response that compromises the mammalian subject's health or that interferes with a therapeutic response in the treated mammalian subject.

"Gene" refers to a nucleic acid sequence encoding a gene product. The gene optionally comprises sequence information required for expression of the gene (*e.g.,* promoters, enhancers, etc.). The term "genomic" relates to the genome of an organism.

"Gene expression data" refers to one or more sets of data that contain information regarding different aspects of gene expression. The data set optionally includes information regarding: the presence of target-transcripts in cell or cell-derived samples; the relative and absolute abundance levels of target transcripts; the ability of various treatments to induce expression of specific genes; and the ability of various treatments to change expression of specific genes to different levels.

"Gene expression profile" refers to a representation of the expression level of a plurality of genes without (*i.e.,* baseline or control), or in response to, a selected expression condition (for example, incubation of the presence of a standard compound or test compound at one or several timepoints). Gene expression can be expressed in terms of an absolute quantity of mRNA transcribed for each gene, as a ratio of mRNA transcribed in a test cell as compared with a control cell, and the like. It also refers to the expression of an individual gene and of suites of individual genes in a subject.

"Isolated" or "purified" refers to altered "by the hand of man" from the natural state *i.e.* anything that occurs in nature is defined as isolated when it has been removed from its original environment, or both. "Isolated" also defines a composition, for example, a mammalian kidney-derived cell population, that is separated from contaminants (*i.e.* substances that differ from the cell). In an aspect, a population or composition of cells is substantially free of cells and materials with which it may be associated in nature. "Isolated" or "purified" or "substantially pure", with respect to mammalian kidney-derived cells, refers to a population of mammalian kidney-derived cells that is at least about 50%, at least about 75%, preferably at least about 85%, more preferably at least about 90%, and most preferably at least about 95% pure, with respect to mammalian kidney-derived cells making up a total cell population. Recast, the term "substantially pure" refers to a population of mammalian kidney-derived cells of the present invention that contain fewer than about 50%, preferably fewer than about 30%, preferably fewer than about 20%, more preferably fewer than about 10%, most preferably fewer than about 5%, of lineage committed kidney cells in the original unamplified and isolated population prior to subsequent culturing and amplification. Purity of a population or composition of cells can be assessed by appropriate methods that are well known in the art.

As used herein, the term "derived" shall also mean obtained. Thus, for example human kidney-derived cells are cells that were isolated and obtained from the human kidney tissue. The term also encompasses cells that were obtained (*e.g*. isolated) from a tissue and then subsequently cultured.

The invention discloses a proximal tubule device comprising a decellularized biological scaffold and a renal proximal tubule cell epithelial monolayer formed from cells that are differentiable into renal cells. The scaffold and cells together create a multi-component, two-dimensional proximal tubule device. This renal proximal tubule cell epithelial monolayer is comprised of functioning proximal tubule cells.

The proximal tubule devices of the invention optimally promote the functioning of natural regulative mechanisms of contact inhibition and the formation of an intact monolayer without use of inhibitors such as *e.g.* MEK inhibitors. Via use of the decellularized biological scaffold, the proximal tubule devices of the invention also advantageously represent and/or mimic the underlying matrix that renal cells are typically exposed to *in vivo.* Optimally, the seeding on a natural decellularized scaffold allows the cells to differentiate and form epithelial monolayers, which are more stable than those produced via traditional methods.

The present invention describes a two-dimensional proximal tubule device that may be used as an *in vitro* testing system for transport studies, renal toxicity screening, or for screening the effects of therapeutic agents. In one embodiment, the two-dimensional bioartificial renal proximal tubule device is constructed by seeding a decellularized biological scaffold with kidney progenitor cells and, optionally, in some instances also microvascular endothelial cells, followed by static culture or culture in a bioreactor to allow differentiation of the kidney cells into functioning proximal tubule cells and maintain the assembled device for *in vitro* testing. These functioning proximal tubule cells form a monolayer on the surface of the scaffold.

The present invention also describes the use of decellularized biological scaffolds as a component of the described bioartificial proximal tubule device. One or more of the surfaces of the decellularized biological scaffold may be seeded with cells. In one embodiment, the decellularized scaffold can be derived from any mammalian tissue, preferably portions of the alimentary canal, most preferably the stomach, duodenum, jejunum, ileum or colon. In one embodiment, the tissue from which the scaffold is derived is most preferably a portion of the jejunum. In one embodiment, the decellularized biological scaffold is derived from mucosal or submucosal tissue. In another embodiment, the decellularized biological scaffold is derived mammalian small intestine submucosa. The decellularized scaffolds, or pieces thereof, may be secured into devices that allow for seeding of each side of the scaffold surface.

The source tissue used for creating the decellularized scaffolds can be any mammalian tissue, including but not limited to human, primate, bovine, sheep, porcine, or rat tissue. In one embodiment of the invention, the tissue is isolated from the mammal. In another embodiment, the decellularized scaffolds are derived from mammalian cell cultures. In yet embodiment, the source tissue is porcine tissue. In preferred embodiments, the scaffolds are derived from porcine tissues isolated from younger animals, such as *e.g*. younger animals less than 6 months old or animals less than 3 months old. In more preferred embodiments, the scaffolds are derived from porcine tissues isolated from younger animals about 10 to 25 kg in weight, alternatively from about 10 to 20 kg in weight, and alternatively from about 15 to about 20 kg in weight. In a most preferred embodiment, scaffolds are derived from porcine tissues isolated from animals about 10 to about 15 kg in weight.

Conventional methods may be used to perform acellularization (*e.g*. decellularization) of the tissue. In one embodiment, the mucosal structures of the isolated tissue are preserved during the isolation. In a preferred embodiment, the mucosal structures are subsequently partially or fully removed to expose the submucosal layer for cell attachment. In one embodiment of the invention, the kidney progenitor cells are cultured on mucosal structures. It was observed that a higher percentage of kidney progenitor cells adopt an epithelial morphology when cultured on the submucosal structures compared to mucosal structures. Accordingly, in an alternate embodiment, the kidney progenitor cells are optimally cultured on submucosal structures.

As used herein, the term "cells differentiable into renal cells" shall mean a precursor cell that differentiate into renal cells *e.g.* any progenitor, precursor, or primary cell that can differentiate into a renal cell. In one embodiment, cells can be selected from primary renal tubule epithelial cells, progenitor (*e.g*. stem) cells differentiated into renal cells or renal progenitor cells (such as *e.g*. inducible pluripotent stem cell), human kidney-derived cells, stem cells isolated from the kidney or progenitor cells isolated from the kidney, and mixtures thereof. Any progenitor (*e.g*. stem) cells that can be differentiated in renal cells or renal progenitor cells may be used including, but not limited to, for example embryonic stem cells, iPS cells, umbilical-derived cells, placental-derived cells or mesenchymal stem cells.

In one embodiment of the invention, the differentiable cells are mammalian kidney-derived cells. Accordingly, one embodiment of the invention is the use of mammalian kidney-derived cells, isolated from the mammal's kidney (such as *e.g.* a human kidney), as a component of the described bioartificial renal system. It has previously been demonstrated, as described in U.S. Pub. App. 2008/0112939 to Colter et al.*,* that progenitor cells can be derived from human kidney tissue and that these kidney-derived cells can self-organize into tubule structure and can be used to treat a diseased kidney.

Exemplary techniques used to isolate, culture, and characterize the mammalian kidney-derived cells are described in U.S. Pub. App. 2008/0112939 to Colter *et al.* As described in U.S. Pub. App. 2008/0112939, human kidney-derived cells are isolated from a human kidney and suitable for organ transplantation. In one embodiment, blood and debris are removed from the kidney tissue prior to isolation of the cells by washing with any suitable medium or buffer such as phosphate buffered saline. The kidney-derived cells, such as *e.g*. human kidney-derived cells, are then isolated from mammalian kidney tissue by enzymatic digestion. Enzymes are used to dissociate cells from the mammalian (*e.g*. human) kidney tissue. In one embodiment, dispase may be used. Alternatively, combinations of a neutral protease (*e.g*. dispase), metalloprotease (*e.g*. collagenase) and hyaluronidase may be used to dissociate cells from the mammalian (*e.g*. human) kidney tissue. Isolated cells are then transferred to sterile tissue culture vessels that are initially coated with gelatin. Mammalian (*e.g*. human) kidney-derived cells are cultured in any culture medium capable of sustaining growth of the cells such as *e.g.,* but not limited to, REGM™ renal epithelial growth medium (Lonza, Walkersville, MD) or ADVANCED™ DMEM/F12 (Invitrogen).

The cells differentiable into renal cells (*e.g*. precursor cells that can differentiate into renal cells) or the mammalian kidney-derived cells may be a population of cells. In one embodiment, a population of human kidney-derived cells are used. In another embodiment, the population is homogenous. In another embodiment, the population is substantially homogenous.

In some embodiments, the kidney-derived cells may be obtained from the kidney cortex, the kidney medulla or the kidney subcapsular region and mixtures thereof.

Mammalian (*e.g*. human) kidney-derived cells are characterized by phenotypic characteristics, for example, morphology, growth potential, surface marker phenotype, early development gene expression, kidney development gene expression and trophic factor secretion. Surface marker, gene expression and trophic factor secretion phenotype is retained after multiple passages of the human kidney-derived cell population in culture.

In preferred embodiments, the isolated mammalian kidney-derived cells (*i.e.* the cell populations) are capable of self-renewal and expansion in culture and exhibit a unique expression profile, such as any of those described below.

In another embodiment of the invention, the human kidney-derived cells are positive for expression of at least one of Oct-4, Rex-1, Pax-2, Cadherin-11, FoxD1, WT1, Eyal, HNF3B, CXC-R4, Sox-17, EpoR, BMP2, BMP7, or GDF5. In yet another embodiment, the cells are negative for the expression of at least one of Sox2, FGF4, hTert, Wnt-4, SIX2 or GATA-4. In an alternate embodiment, the cells are positive for expression of at least one of Oct-4, Rex-1, Pax-2, Cadherin-11, FoxD1, WT1, Eyal, HNF3B, CXC-R4, Sox-17, EpoR, BMP2, BMP7, or GDF5 and negative for the expression of at least one of Sox2, FGF4, hTert, Wnt-4, SIX2 or GATA-4. In an alternate embodiment, the cell is positive for expression of at least one of Eyal, WT1, FoxD1, BMP7, BMP2, GDF5, EpoR or Rex-1. In yet another alternate embodiment, the cells are negative for expression of at least one of Sox2, FGF4, hTert or Wnt-4. In an alternate embodiment, the cells are positive for expression of at least one of Eyal, WT1, FoxD1, BMP7, BMP2, GDF5, EpoR or Rex-1, and negative for expression of at least one of Sox2, FGF4, hTert or Wnt-4. In one embodiment of the invention, the human kidney-derived cells are also positive for at least one of cell-surface markers HLA I, CD24, CD29, CD44, CD49c, CD73, CD166, or SSEA-4. In another embodiment, the human kidney-derived cells are also negative for at least one of cell-surface markers HLA II, CD31, CD34, CD45, CD56, CD80, CD86, CD104, CD105, CD117, CD133, CD138, CD141, or E-cadherin. In an alternate embodiment, the human kidney-derived cells are also positive for at least one of cell-surface markers HLA I, CD24, CD29, CD44, CD49c, CD73, CD166, or SSEA-4, and negative for at least one of cell-surface markers HLA II, CD31, CD34, CD45, CD56, CD80, CD86, CD104, CD105, CD117, CD133, CD138, CD141, or E-cadherin. In one embodiment, the human kidney-derived cells may secrete at least one of the trophic factors FGF2, HGF, TGFα, TIMP-1, TIMP-2, MMP-2 or VEGF. In a preferred embodiment, the cells do not secrete at least one of trophic factors PDGFbb and IL12p70.

In an alternate embodiment, the progenitor cells used with the bioartificial proximal tubule device are human kidney-derived cells. These human kidney-derived cells are capable of self-renewal and expansion in culture and are positive for expression of at least one of Oct-4, Rex-1, Pax-2, Cadherin-11, FoxD1, WT1, Eyal, HNF3B, CXC-R4, Sox-17, EpoR, BMP2, BMP7, or GDF5; and negative for the expression of at least one of Sox2, FGF4, hTert, Wnt-4, SIX2, E-cadherin or GATA-4. Furthermore, the human kidney-derived cells may also be positive for at least one of cell-surface markers HLA-I, CD24, CD29, CD44, CD49c, CD73, CD90, CD166, or SSEA-4; and negative for at least one of cell-surface markers HLA II, CD31, CD34, CD45, CD56, CD80, CD86, CD104, CD105, CD117, CD133, CD138, and CD141. In addition, these cells optionally cells secrete at least one of trophic factors FGF2, HGF, TGFα, TIMP-1, TIMP-2, MMP-2 or VEGF; and do not secrete at least one of trophic factors PDGF-bb or IL12p70.

In yet another embodiment, the human kidney-derived cells are (1) positive for expression of Oct-4, Rex-1, Pax-2, Cadherin-11, FoxD1, WT1, Eyal, HNF3B, CXC-R4, Sox-17, EpoR, BMP2, BMP7 and GDF5 and (2) negative for the expression of at least one of Sox2, FGF4, hTert, SIX2 and Gata-4. In an alternate embodiment, the kidney-derived cells are (1) positive for expression of Oct-4, Rex-1, Pax-2, Cadherin-11, FoxD1, WT1, Eyal, HNF3B, CXC-R4, Sox-17, EpoR, BMP2, BMP7 and GDF5; (2) negative for the expression of at least one of Sox2, FGF4, hTert, SIX2 and Gata-4; (3) positive for cell-surface markers HLA I, CD24, CD29, CD44, CD49c, CD73, CD166, and SSEA-4; and (4) negative for HLA II, CD31, CD34, CD45, CD56, CD80, CD86, CD90, CD104, CD105, CD117, CD133, CD138, CD141, and E-cadherin.

In another embodiment, the human kidney derived cells are capable of self-renewal and expansion in culture, positive for the cell surface marker expression HLA I and CD44, positive for the gene expression of Oct-4, Pax-2, and WT1, negative for the cell surface marker expression of CD133 and the gene expression of Wtn-4. In one embodiment, the human kidney derived cells are additionally positive for the gene expression of BMP7, BMP2, GDF4, EpoR and Rex-1, and negative for the gene expression of Sox2, FGF4 and hTert.

In an alternate embodiment, the human kidney-derived cells are: (1) capable of self-renewal and expansion of culture; (2) positive for the expression of HLA-I and at least one of Oct-4, Rex-1, Pax-2, Cadherin-11, FoxD1, WT1, Eyal, HNF3B, CXC-R4, Sox-17, EpoR, BMP2, BMP7 or GDF5; and (3) negative for the expression of CD133 and at least one of SOX2, FGF4, hTert, Wnt-4, SIX2, E-cadherin or GATA-4. These cells may further be (4) positive for at least one of the cell surface markers CD24, CD29, CD44, CD49c, CD73, CD90, CD166 or SSEA-A and (5) negative for at least one of the cell-surface markers HLA II, CD31, CD34, CD45, CD56, CD80, CD86, CD104, CD105, CD117, CD133, CD138, CD141, and E-cadherin. These cells may also secrete at least one of the trophic factors FGF2, HGF, TGFα, TIMP-1, TIMP-2, MMP-2 or VEGF and lack secretion of at least one of trophic factors PDGFbb or IL12p70. In an alternate embodiment, the human kidney-derived cells are a population.

Mammalian (*e.g*. human) kidney-derived cells are passaged to a separate culture vessel containing fresh medium of the same or a different type as that used initially, where the population of cells can be mitotically expanded. The mammalian (*e.g*. human) kidney-derived cells are then seeded into the biological matrix, and cultured to allow differentiation of the kidney-derived cells into functioning proximal tubule cells. The cells of the invention may be used at any point between passage zero and senescence. The cells preferably are passaged between about 3 and about 20 times, between about 5 and about 10 times, between about 15 and 20 times, between about 5 and about 7 times, and more preferably between about 3 and about 7 times.

In one embodiment of the invention, two or more surfaces of the decellularized biological scaffold are seeded with cells. In one embodiment, vascular endothelial cells are seeded on one surface of the scaffold, and the other surface is then seeded with mammalian kidney-derived cells. The seeded scaffold is then cultured to allow differentiation of the kidney-derived cells into functioning proximal tubule cells and the formation of a vascular endothelial monolayer on the opposing surface.

In one embodiment, the human vascular endothelial cells used for repopulation of the scaffold can be selected from endothelial cell lines, bone marrow or whole blood endothelial progenitor cells, or primary endothelial or microvascular endothelial cells. The vascular endothelial cells used are isolated using conventional methods. In a preferred embodiment, the cells used for repopulation of the scaffold are primary microvascular endothelial cells. In one embodiment, the vascular endothelial cells used can be isolated from any mammalian source. In a preferred embodiment, the vascular endothelial cells isolated are of human origin. In an alternate preferred embodiment, the vascular endothelial cells are primary microvascular endothelial cells isolated from a mammalian (human) kidney.

Another embodiment of the invention is an apparatus for making the proximal tubule devices.

In one embodiment, seeding of the decellularized scaffolds is accomplished by using a specially designed apparatus ("crown") in which a piece of the decellularized scaffold is inserted so its edges are placed between two pieces of metal or plastic, effectively sealing the edges to create an upper and lower well separated by the scaffold. The crown also introduces some stretch and tension into the decellularized scaffold. Cells are then seeded into the upper well and allowed to settle onto the decellularized scaffold. The crown can also be flipped over to allow seeding of the other side of the scaffold, or the crown and be disassembled, the scaffold turned over and reassembled into the crown for seeding of the opposite surface. The renal cells may be seeded onto the scaffolds at a density ranging from about 500 cells/cm² to about 350,000 cells/cm², alternatively from about 1,000 cells/cm² to about 100,000 cells/cm², alternatively from about 750 cells/cm² to about 75,000 cells/cm², alternatively from about 10,000 cells/cm² to about 300,000 cells/cm², alternatively from about 7,500 cells/cm² to about 200,000 cells/cm² and preferably from about 5,000 cells/cm² to about 70,000 cells/cm².

The cell-seeded scaffold with the apparatus is then cultured using conventional techniques to allow for differentiation of the renal cells and the formation of a continuous epithelial monolayer. The time of culture may be from 1 to 6 weeks of culture, preferably 2 to 4 weeks of culture, most preferably 3 to 4 weeks. The resulting mature proximal tubule device can then be used for renal transport studies, nephrotoxicity testing, or testing of therapeutic agents using conventional methods.

In another embodiment, the culture of cells on the seeded scaffold, as well as the *in vitro* test system, is achieved by placing the scaffolds in a custom designed bioreactor such that the scaffold creates a barrier between two compartments. The bioreactor chamber is connected to a fluid flow system designed to allow fluid flow across both surfaces of the scaffold. By altering the flow system characteristics, such as flow rates, cell specific fluid mechanical conditions can be established for each side of the scaffold, depending on the cell type seeded, for example, to support the functionality of endothelial cells at the basolateral side. The scaffolds may be pre-seeded with cells before placement into the bioreactor, or placed into the bioreactor followed by seeding of cells within the bioreactor. In another aspect, the bioreactor is designed in a way such that the tension applied to the decellularized construct placed within the bioreactor can be altered as needed to facilitate the seeding and/or differentiation of cells.

In one embodiment, the decellularized scaffold is seeded with cells using the apparatus described for seeding of renal cells and, optionally, endothelial cells. The cell-seeded scaffolds are then removed from the apparatus and transferred to the bioreactor chamber for culture or assessments, as described below in the examples. The cell-seeded scaffolds may be first cultured within the apparatus for a period of approximately 0 to 4 weeks before transfer to the bioreactor chamber. The renal cells may be seeded onto the scaffolds at a density ranging from about 500 cells/cm² to about 350,000 cells/cm², preferably about 5000 cells/cm² to about 70,000 cells/cm². The culture conditions, including the duration of incubation in the apparatus, may vary depending on the source of the cell and the culture medium.

In another embodiment, the decellularized scaffolds are first placed within a bioreactor chamber, and then scaffolds are seeded by perfusing a cell suspension into the bioreactor chamber and incubating for a period of time to allow cell attachment to the decellularized scaffold. Such a bioreactor comprises an upper body element and a lower body element having an area designated for scaffold growth.

One exemplary bioreactor suitable for use in the instant invention is shown in Figure 15. With reference to Figure 15, the components of bioreactor 100 are the main body elements, upper body element 110 and lower body element 120, two clips, front clip 130 and back clip 160, the outer closure flaps, lower outer closure flap 140 and upper outer closure flap 150 and the connectors 170. The main reactor upper body element 110 and lower body element 120 of bioreactor 100 are held together by front clip 130 and back clip 160. Upper outer closure flap 150 is on top of the upper body element 110. Lower outer closer flap 140 is below the lower body element 120. Figure 16 is a view of the lower body element 120 of the bioreactor showing the groove 180 whose depth can be altered along with a frame in upper body element to adjust tension on the decellularized scaffold. Lower body element 120 also contains the area for cell growth 190.

In one embodiment, the unseeded scaffold can be positioned between upper body element 110 and lower body element 120 of the bioreactor 100 (*see* Figure 15). A circular frame construction, which is milled into upper body element 110 with a corresponding groove structure in lower body element 120, allows the fixation of the scaffold comparable to the cell crowns. The tension can be adjusted by using specifically designed frame/groove combinations, which differ in depth of the groove and bridge width of the frame. The distance which the frame can be moved into the groove 180 (*see* Figure 16), determines the tension of the scaffold, with a longer distance leading to a higher tension. Factors like scaffold diameter and scaffold thickness must be considered. After scaffold positioning, front clip 130 and back clip 160 of the bioreactor keep upper body element 110 and lower body element 120 together and the construct can be handled like a cell crown. Lower outer closure flap 140 and upper outer closure flap allow closure of the system and a cell suspension can be introduced to one side of the scaffold via the connectors 170 of the bioreactor. The cells grow in cell growth area 190. After a cell-specific time period, in which the seeded cells may adhere, the closed bioreactor can be flipped over and a second or the same cell type can be seeded on the other side of the scaffold. In one embodiment, the cell suspensions used for seeding may range from about 10³ cells/ml to about 10⁷ cells/ml. In order to avoid a loss of viability of the cells seeded on the first side, the compartment of the first side can be filled with media. If static conditions are required the compartment can be filled with cell culture media. Alternatively, perfusion of media under various flow conditions can be initiated using the connectors 170.

After adhesion of the cells to the scaffold, the cells can be cultured statically by simply flooding the chamber compartments with media and closing the connectors of the bioreactor. Alternatively, tubes of a flow system are connected to the bioreactor and perfusion can be started. Cells seeded onto decellularized scaffolds within the bioreactor chambers are then cultured to allow for growth and differentiation of the cells into a functional monolayer of renal tubular cells. Culture of the cells may include static culture, or more preferably culture under dynamic conditions such as linear flow or pulsatile flow. Flow rates, pressure, and pulsatile conditions may all be varied to facilitate the growth and differentiation of the cells into functional renal cells. The mean flow rate of media within the bioreactor may range from 1 to 25 ml/min, alternatively from about 1 to about 10 ml/min, alternatively from about 2.5 to about 10 m/min, alternatively from about 5 to 20 ml/min. In a preferred embodiment, the mean flow rate may range from about 2.5 to 15 ml/min. The culture period may range from about 1 to about 4 weeks, alternatively from about 1 to about 2 weeks, alternatively from about 1 to about 3 weeks, alternatively from about 2 to about 4 weeks. In a preferred embodiment, the culture period may range from preferably about 2 to 3 weeks. During this time, the cell layer integrity can be monitored using techniques well-known in the art. In one embodiment, the cell layer integrity can be monitored by measuring the trans-epithelial electrical resistance using electrodes integrated in each compartment of the bioreactor or by measuring leakage across the monolayer of various fluorescently tagged molecules, such as *e.g.* inulin or creatinine. In another embodiment, different cell culture media are used in each chamber. In yet another embodiment, different flow rates, pressure, and pulsatile conditions may be used in each chamber allow cell specific cultivation via flow indices shear stress. A description of the bioreactor can also be found in Patent Application No. DE 102008056037.5-41 and EP 2 184 344.

Another embodiment of the invention is a method of differentiating the cells differentiable into renal cells (such as *e.g.* the mammalian (human) kidney-derived) cells into a stable monolayer of renal proximal tubule cells by differentiating the cells under tension. This method may further comprise the use of the scaffolds of the invention to produce proximal tubule devices of the invention.

The proximal tubule devices of the invention may be used as *in vitro* testing systems for renal toxicity screening or for screening of therapeutic agents. In another embodiment, the devices may be used to monitor tubule cell function, such as transport, during or after exposure to a compound or particle. Different media formulations can be used for the flow over each surface, allowing one to study transport across the cell and scaffold layers from one media compartment to the other. One example of such media formulations would be to flow an endothelial cell media in the compartment that had the mvECs seeded on it, and to flow a media formulation that mimics the glomerular filtrate in the opposite compartment that contains the renal tubular monolayer.

Transport functions of the renal tubular monolayer can then be assessed using standard techniques and labeled molecules known by those skilled in the art. Toxicity screening can be achieved by adding compounds or particles of interest to either the vascular compartment flow path that provides nutrients to and contacts the endothelial cells or by introducing compounds or particles to the tubule compartment flow path that provides nutrients to and contacts the renal tubular cells, mimicking the appearance of toxic xenobiotic compounds in the blood and urine, respectively. Transport of compounds or particles can be monitored by assaying the medium of one or both of the flow paths. In addition, toxicity can be monitored by assaying the cell viability, morphology, or effect on transport functions after exposure to the compounds of interest. Assays used for the assessment of toxicity or therapeutic effects of compounds or particles are not limited to those described above.

Therapeutic targets can be assayed by first injuring the kidney-derived cells of the bioartificial device, then treating the cells with the test therapeutic particle. The injury can be introduced by physical or chemical means, such as *e.g.* exposing the cells to toxic compounds or particles, such as *e.g.* cisplatin or streptozotocin. Test therapeutic compounds or particles can be put in contact with the cells by addition into the vascular compartment flow path of the device, for example, in a concentration that would mimic the concentrations cells would be exposed to after intravenous (IV) delivery of the therapeutic agent. Monitoring of renal tubular cell function can then be used to determine the degree of effectiveness of the applied test therapeutic compounds.

Monitoring tubular function includes, but is not limited to, detecting or assessing the uptake of a compound or particle by renal tubular cells, transport of a compound or particle taken up by the cells from one media compartment of the proximal tubule devices to the other, the effect of inhibitors on uptake or transport, and changes in renal tubule cell gene or protein expression, morphology, surface marker expression, enzymatic activity or survival. Assays used for the assessment of toxicity or therapeutic effects of compounds or particles are not limited to those described above.

Another embodiment of the invention is kits comprising the bioartificial proximal tubule devices of the invention. In one embodiment, the kit comprises the bioartificial tubule device and a product insert.

An alternate embodiment of the invention is a composition comprising the bioartificial proximal tubule devices of the invention. In one embodiment, the composition comprises a bioartificial proximal tubule device of the invention and a pharmaceutically acceptable carrier.

Without further description, it is believed that one of ordinary skill in the art can, using the preceding description and the following illustrative examples, make and utilize the present invention and practice the claimed methods. The following working examples therefore, specifically point out the preferred embodiments of the present invention, and are not to be construed as limiting in any way the remainder of the disclosure. Additionally as used in the following examples and elsewhere in the specification, the kidney-derived cells ("hKDC") useful in the devices and methods of the invention may be isolated and characterized according to the disclosure of U.S. Patent Publication No. 2008/0112939, as it relates to the description, isolation and characterization of hKDC.

### Examples

### Example 1: Seeding and differentiation of hKDC on extracellular matrix scaffolds

This experiment tests the attachment, growth and differentiation of human kidney-derived cells ("hKDC") on various configurations of extracellular (*i.e* decellularized) matrix scaffolds as well as to the traditional culture on collagen-coated transwells.

hKDC at passage 4 were seeded onto three different scaffold configurations and on transwells (Corning, Corning NY). The cells were cultivated over a time period of three weeks with REGM™ renal epithelial growth medium (Lonza, Walkersville MD). Each scaffold configuration was tested with three different cell concentrations: 2.5 x 10³, 5 x 10³, and 1x10⁴ cells. The configurations tested were: 1) collagen sandwich culture; 2) collagen-SIS sandwich culture; 3) SIS monolayer culture; and 4) collagen-coated transwells.

### Collagen Sandwich Cultures

hKDC were cultivated between two collagen gel layers in 24-well plates. The bottom gels were cast by first mixing a cold gel neutralization solution with collagen (type 1 isolated from rat tail tendons, 6 mg/ml in 0.1 % acetic acid) in a 1:2 ratio and then adding 500 µl of this solution per well. The solution was gelled by incubation at 37 °C / 5 % CO₂ for 15 minutes. Afterwards, the cells were seeded in 1 ml medium per well. After 24 hours, the cover gels were cast. The medium was aspirated and 300 µl of gel solution (prepared as above) was pipetted into each well. The solution was gelled by incubation for 15 minutes at 37 °C / 5 % CO₂. Finally, 1 ml medium was added per well.

### Small Intestine Submucosa (SIS) Monolayer Cultures

Scaffolds were prepared by decellularizing segments of small intestine submucosa (SIS) using conventional methods. Briefly, the mucosa of segments of porcine small intestine was removed mechanically. Afterwards, decellularization was performed by incubating the intestinal segments in 3.4% sodium deoxycholate for 1 hour at 4 degrees Celsius with shaking. The decellularized segments were then rinsed extensively with PBS and sterilized by gamma irradiation. 12-well plates were used for the SIS monolayer cultures. Decellularized SIS was spanned over round metal frames and covered with 1 ml medium per well the day before seeding the cells. The spanned SIS surface is approximately equivalent to the surface of a well in a 24-well plate. To seed the cells onto the SIS, cell culture medium was removed and the appropriate amount of cells was seeded in 1 ml of medium per well.

### SIS Sandwich Cultures

SIS scaffolds were prepared and seeded with hKDCs as described above. After allowing the cells to attach for 24 hours, a cover gel was cast over the cells as in the collagen sandwich cultures (*see* above).

### Transwell Cultures

Transwells were transferred into 12 well plates and coated, each with 200 µl collagen type 1 (100 µg/ml in 0.1 % acetic acid). The coating solution was incubated for 20 minutes at room temperature and then aspirated. The appropriate amount of cells was seeded in 1.5 ml medium per well.

Multiple samples were cultured for three weeks in each of the above conditions. Samples were removed for histological analysis after one, two, and three weeks. In addition, media samples were analyzed with a RANDOX RX DAYTONA™ clinical analyzer for following the metabolic parameters: glucose concentration, lactate and lactate dehydrogenase. The samples taken for histological analysis were fixed with Bouin's fixative for 1 hour. Then they were washed in water for at least 4 hours and embedded into paraffin. Subsequently, 3 µm thick slices were prepared. The slices were stained with hematoxylin and eosin (H&E) to assess cell morphology. Furthermore, immunohistochemistry (IHC) was performed to characterize the differentiation of the cells using the following antibodies: Anti-hPAX2, Anti-hAQP1, Anti-Ki67, Anti hE-cadherin and Anti-hN-cadherin (*see* Table 1 below). For the immunohistochemistry, 3 µm cross sections were deparaffinized. Target retrieval was achieved by either enzymatic treatment with proteinase K or by heating in citrate buffer pH 6 (Dako, #S2369) or Tris/EDTA buffer pH9 (Dako, #S2367). A blocking step with 3% hydrogen peroxidase was included to block endogenous peroxidases. Primary antibodies were then incubated for 1 hour followed by their detection with the ENVISION™ Detection System Peroxidase/DAB Rabbit/Mouse (Dako, #K5007). Slices were counterstained with hematoxylin.

| **Table 1: Antibodies used for Immunohistochemistry** | | | |
|---|---|---|---|
| Antigen | Host | Distributor | Catalog # |
| Aquaporin 1 | mouse | Abcam | Ab9566 |
| Aquaporin 2 | rabbit | Abcam | Ab15081 |
| E-Cadherin | mouse | BD Biosciences | 610181 |
| Ki67 | mouse | Dako | M7240 |
| N-Cadherin | rabbit | Abcam | Ab12221 |
| Pax-2 | rabbit | Invitrogen | 71-6000 |

The results from the analysis of metabolic parameters indicated that the hKDCs seeded well and proliferated under all culture conditions tested (*see* Figure 1 to 6). Histological staining with H&E showed that in both the collagen sandwich and SIS-collagen sandwich culture conditions, the cells grew into nearly continuous double or multiple layers, which progressed into three-dimensional structures resembling tubes or cysts. In contrast, the cells cultured on the SIS formed a confluent monolayer which displayed a highly prismatic morphology which is indicative of an epithelial differentiation (*see* Figures 7 and 8). This morphology was observed after three weeks of culture regardless of the initial cell seeding concentration used. hKDC cultured on transwells with collagen-coated PET membranes show a flat morphology, multilayer formation and agglomerates that were observed to peel off the surface, which leads to a non-continuous cell layer. These observed properties of the hKDC on collagen-coated PET membranes make them unsuitable for transport assays. Similar multi-layering has also been observed with polyester and polycarbonate transwell membranes, indicating that this effect is a property of synthetic membranes in general, not the specific membranes used in the experiment. These properties were not observed for the SIS culture, which implies that this scaffold promotes the functioning of natural regulative mechanisms of contact inhibition and the formation of an intact monolayer.

Immunohistochemistry results using Ki67 antibodies indicate that the hKDCs are proliferating in each of the scaffold configurations tested. Expression of the kidney transcription factor Pax-2 was also positive throughout the culture period. Cadherins as constituents of desmosomes and adherens junctions are involved in cell-cell contacts and their expression is a marker for cellular differentiation. They are essential for the polarization of cells and thus for their functionality. In the kidney, N-Cadherin is expressed by proximal tubule cells whereas E-Cadherin is prevalent in distal tubule cells. IHC results show strong immunostaining for N-Cadherin by the hKDCs in all scaffold configurations. In contrast, immunostaining for E-cadherin was largely absent, although there were few areas of extremely weak staining observed.

Aquaporins catalyze the transport of water through the cell membrane and are thus very important for kidney functionality. Aquaporin 1 is expressed by proximal kidney cells whereas Aquaporin 2 is predominant in distal tubule cells. Aquaporin 1 expression could be detected after 2 and 3 weeks of culture whereas Aquaporin 2 expression was not observed. In areas, the staining of Aquaporin-1 was observed to be only on the apical side of the cuboidal cells (*see* Figure 9) again indicating a mainly proximal differentiation of the cells. IHC of sodium glucose co-transporter 1 (SGLT-1), which is a transporter expressed by distal tubule cells, was also assayed, but no marker expression could be observed in any of the samples.

This example demonstrates that hKDCs are able to differentiate into proximal tubule cells, and that this differentiation is dependent on the composition of the substrate onto which the cells are seeded. A planar natural extracellular (*i.e.* decellularized) matrix outperformed collagen, SIS/collagen scaffolds as well as standard transwell culture with regard to hKDC epithelial morphology and differentiation. Non-transformed proximal tubule cells (such as primary cells) typically will continue to grow once confluence is reached, resulting in the formation of three-dimensional aggregates on synthetic planar surfaces such as transwells. Seeding on a natural decellularized scaffold, such as SIS, allows the cells to differentiate and form epithelial monolayers that are more stable than those produced via traditional methods.

### Example 2: Optimization of cell seeding concentration of hKDC on decellularized scaffolds

Example 1 demonstrated that cells seeded onto two-dimensional decellularized scaffolds without collagen formed a confluent epithelial monolayer expressing proximal tubule markers after three weeks of culture. The following experiments were conducted to optimize the cell seeding density and attempt to reduce the culture period necessary for formation of the monolayer.

Scaffolds were prepared by decellularizing segments of small intestine submucosa (SIS) as described in Example 1. hKDC at passage 4 were seeded onto the SIS scaffolds at three different concentrations (1 x 10⁴, 5 x 10⁴, and 1 x 10⁵ cells/well) and cultivated for three weeks with REGM™ renal epithelial growth medium (Lonza, Walkersville). Samples were removed for histological analysis after two and three weeks and were fixed with Bouin's fixative for 1 hour. Thereafter they were washed in water for at least 4 hours and embedded into paraffin. Subsequently, 3 µm thick slices were prepared. The slices were stained with hematoxylin and eosin (H&E) to assess cell morphology. In addition, immunohistochemistry (IHC) was performed, as in Example 1, to characterize the differentiation of the cells. First, 3 µm cross sections were deparaffinized. Target retrieval was achieved by enzymatic treatment with proteinase K, by heating in citrate buffer pH 6 (Dako, #S2369) or by heating in Tris/EDTA buffer pH 9 (Dako, #S2367). A blocking step with 3 % hydrogen peroxidase was included to block endogen peroxidases. Primary antibodies were then incubated for 1 hour followed by their detection with the ENVISION™ Detection System Peroxidase/DAB Rabbit/Mouse (Dako, #K5007). The slices were counterstained with hematoxylin.

Lectin staining was performed to further evaluate cell differentiation. As described above, 3 µm cross sections were deparaffinized and blocked with hydrogen peroxidase. Biotinylated lectins, either *Lotus tetragonobolus* lectin (Biozol, #B-1325) or *Dolichos biflorus* agglutinin (Biozol, #B1035), were then incubated for 1 hour, followed by labeling with streptavidin (Biogenex, #LP000-ULE) and detection by addition of aminoethyl carbazole chromogen (AEC) (Biogenex, #HK129-5KE). Slices were counterstained with hematoxylin.

Results of the H&E staining indicated that after two weeks of culture, none of the seeding densities examined had reached 100% confluence. However, after three weeks of culture, the cells seeded at all densities had formed a nearly confluent monolayer and had areas which developed a typical cuboidal epithelial morphology with nuclei located in the lower third of the cell. The cells seeded at the higher density were also nearly confluent, but the morphology of the cells was less uniform and not as epithelial in appearance (*see* Figure 10).

Immunohistochemistry results are summarized in Table 2 below. IHC of the lower seeding density three week samples detected expression of proximal tubules markers AQP-1 (40-50 %) and N-Cadherin (∼90 %) whereas the distal markers were not (AQP-2 and SGLT-1) or only very weakly (E-Cadherin) expressed. Also, PAX-2 expression was detected throughout the sample, and Claudin-2 a tight junction marker, was detected in several regions of the samples. Ki67, a marker of proliferation, was only detected in areas where the monolayer was not completely confluent. Importantly, basolateral staining of collagen IV, a basement membrane protein was observed in many areas of the samples. Expression of collagen IV indicated that the cells were remodeling the scaffold and depositing a new basement membrane.

Staining of the lower seeding density three week samples with the biotinylated lectins *Lotus tetragonobulus* lectin (LTL), a marker of proximal tubule cells, and *Dolichos biflorus* agglutinin (DBA), a marker of distal tubule cells, also indicated that the major portion of the cells differentiated into proximal tubule cells. Expression of LTL was observed in many areas of the samples, whereas expression of DBA was sparse and weak (*see* Figure 10).

This example demonstrates that the degree of differentiation of the cells is dependent on the seeding density. However, unexpectedly, in contrast to what would normally be assumed, a lower seeding density resulted in a greater degree of differentiation.

The sample seeded at 1 x 10⁴ cells/well was also analyzed for the expression of collagen type IV by immunohistochemistry, as described above, using anti-human collagen IV antibody (Dako, #M0785). The results, shown in Figure 11, demonstrate positive staining of collagen IV on the basolateral surface of the cells, indicating that the cells were actively secreting extracellular matrix components on the decellularized natural scaffold; demonstrating that intact monolayers can be developed on decellularized natural scaffolds, which are more physiologically relevant than synthetic scaffolds, such as transwells. Further, it suggests that the cell seeding density has a direct effect on cell differentiation and monolayer formation.

| **Table 2: Immunohistochemistry results in relation to initial seeding density after various time periods of culture.** | | | | | | | | | | | | | | | | | | |
|---|---|---|---|---|---|---|---|---|---|---|---|---|---|---|---|---|---|---|
| | N-Cadherin | | | E-Cadherin | | | APQ-1 | | | Ki67 | | | LTL | | | DBA | | |
| | 10⁴ | 5^{∗}10⁴ | 10⁵ | 10⁴ | 5^{∗}10⁴ | 10⁵ | 10⁴ | 5^{∗}10⁴ | 10⁵ | 10⁴ | 5^{∗}10⁴ | 10⁵ | 10⁴ | 5^{∗}10⁴ | 10⁵ | 10⁴ | 5^{∗}10⁴ | 10⁵ |
| 7 d | +++ | +++ | +++ | +/- | +/- | +/- | - | +/- | +/- | + | + | + | ++ | + | + | +/- | +/- | +/- |
| 14 d | +++ | +++ | +++ | +/- | +/- | +/- | + | + | + | +/- | +/- | +/- | ++ | + | + | +/- | +/- | + |
| 21 d | +++ | +++ | +++ | +/- | +/- | +/- | + | +(+) | + | - | - | - | ++ | ++ | + | +/- | +/- | + |
| Legend: | | | | | | | | | | | | | | | | | | |
| - = no detection | | | | | | | | | | | | | | | | | | |
| +/- = < 10% positive | | | | | | | | | | | | | | | | | | |
| ++ = 40-80% positive | | | | | | | | | | | | | | | | | | |
| +++ = 90-100% positive | | | | | | | | | | | | | | | | | | |
| AQP-1 =Aquaporin-1 | | | | | | | | | | | | | | | | | | |
| KI = Ki67 | | | | | | | | | | | | | | | | | | |
| LTL = *Lotus tetragonobulus* lectin | | | | | | | | | | | | | | | | | | |
| DBA = *Dolichos biflorus* agglutinin | | | | | | | | | | | | | | | | | | |

### Example 3: Immunohistochemistry of p-glycoprotein-1

Scaffolds were prepared by decellularizing segments of small intestine submucosa (SIS) as described in Example 1. hKDC at passage 4 were seeded onto the SIS scaffolds at 5 x 10⁴ cells/scaffold and cultivated for three weeks with REGM™ renal epithelial growth medium (Lonza, Walkersville). Samples were removed for histological analysis after three weeks and fixed with Bouin's fixative for 1 hour. Afterwards, they were washed in water for at least 4 hours and embedded into paraffin. Subsequently, 3 µm thick slices were prepared. Immunohistochemistry (IHC) was performed to confirm the expression of p-glycoprotein-1 (pgp-1 aka MDR1), which is an efflux transporter expressed by proximal tubule cells. Target retrieval of deparaffinized sections was achieved by enzymatic treatment with proteinase K, by heating in citrate buffer pH 6 (Dako, #S2369), or by heating in Tris/EDTA buffer pH 9 (Dako, #S2367). A blocking step with 3 % hydrogen peroxidase was included to block endogen peroxidases. Primary anti-human pgp-1 (Biogenex, #AM-391-5M) was then incubated for 1 hour followed by their detection with the ENVISION™ Detection System Peroxidase/DAB Rabbit/Mouse (Dako, #K5007). Slices were counterstained with hematoxylin.

Results showed positive staining for pgp-1 on the apical membrane of the cellular monolayer (*see* Figure 13), confirming expression of a functional marker of proximal tubule cells, further indicating that the scaffold and seeding methods described allow for differentiation of hKDCs into functioning proximal tubule cells.

### Example 4: Albumin uptake by hKDCs seeded on decellularized scaffolds

Scaffolds were prepared by decellularizing segments of small intestine submucosa (SIS) as described in Example 1. hKDC at passage 4 were seeded onto the SIS scaffolds at 5 x 10⁴ cells/scaffold and cultivated for three weeks with REGM™ renal epithelial growth medium (Lonza, Walkersville). To assess albumin uptake, cell-seeded samples were first pre-incubated in serum-free medium (REBM basal medium, Lonza, Walkersville) for 1 hour. The media was then exchanged with REBM basal medium containing 200 µg/ml fluorescently labeled bovine serum albumin (BSA-FITC) (Sigma, #A9771) and incubated for 30 to 60 minutes. The samples were then washed with PBS, counterstained with DAPI (diamidino-2-phenylindole) and imaged on a fluorescent microscope.

The results showed that cells are able to take up BSA-FITC (*see* Figure 14), a function of proximal tubule cells. These results demonstrated that hKDCs seeded onto decellularized scaffolds not only express markers of renal proximal tubule differentiation but also function as proximal tubule epithelial cells.

Examples 5 to 9 that follow are prophetic Examples designed to further elucidate properties for the scaffolds of the invention. Functional assays, such as BSA-FITC uptake, can be used to assess cellular injury or nephrotoxicity, as well as the effectiveness of therapeutic compounds on the restoration of renal transport functions.

### Example 5: Organic anion transport by hKDCs seeded on decellularized scaffolds

This example tests function of organic anion transporters, as well as pgp-1, an efflux transporter, by assaying transport of fluorescent dyes, such as rhodamine, lucifer yellow, or carboxyfluorescein. Transport into the cell is mediated by various organic anion transporters (OATs). When the pgp-1 transporter is inhibited by verapamil, applied dye such as rhodamine ceases to be transported out of the cell, resulting in an increase in cellular fluorescence.

Scaffolds will be prepared by decellularizing segments of small intestine submucosa (SIS) as described in Example 1. hKDC at passage 4 will be seeded onto the SIS scaffolds at 5 x 10⁴ cells/scaffold and cultivated for three weeks with REGM™ renal epithelial growth medium (Lonza, Walkersville). The media in the top compartment will then be exchanged with phenol-red free medium containing various concentrations of rhodamine, lucifer yellow or carboxyflourescein dye. Some wells will also be pre-incubated with various concentrations of verapamil in both compartments prior to the addition of the media containing the fluorescent dye (with and without additional verapamil in the medium) to the top compartment. The wells will then be incubated for 30 to 120 minutes. The samples will be then washed with PBS, fixed, counterstained with DAPI and imaged on a fluorescent microscope. In addition, samples of the medium will be taken for quantitative analysis of the presence of fluorescent dye.

### Example 6: Seeding and differentiation of hKDCs (with or without microvascular endothelial cells) on decellularized scaffolds within a flow bioreactor.

The bioreactor system will be prepared as described above. The scaffold is positioned between upper body element 110 and lower body element 120 of bioreactor 100 and fixated with a tension comparable to the cell crowns (*see* Figure 15). The tension on the scaffold positioned in the bioreactor can be altered and experiments will be conducted to determine the tension ranges most appropriate for formation of a monolayer of cells and subsequent differentiation. One side of the scaffold will be seeded with hKDCs followed by an appropriate adherence period (without flow). The other side of the scaffold may then be seeded with endothelial cells. As a control, there will also be bioreactors with only one of the cell types. The bioreactor chambers will then be perfused after an appropriate period of cell adhesion. The flow rate will be adjusted to renal conditions to promote differentiation and epithelial monolayer formation. Monolayer formation and integrity will be monitored by periodic measurement of trans-epithelial electric resistance (TEER) as well as by measuring leakage of fluorescent FITC-inulin. In cases where both cell types are used, the flow conditions will be adjusted to those appropriate for maintenance of both epithelial and endothelial cell monolayers. In addition to TEER and inulin monitoring, samples will be fixed after 1, 2 and 3 weeks. Epithelial morphology and monolayer formation will be evaluated by hematoxylin and eosin staining.

### Example 7: Functional transport of glucose and reabsorption of other solutes by hKDCs (with or without microvascular endothelial cells) seeded on SIS scaffolds and cultured within a flow bioreactor

This example demonstrates functional differentiation of hKDCs into proximal tubule epithelial cells when seeded onto SIS scaffolds in a flow bioreactor by analyzing active transport of glucose and other solutes from one media compartment across the cell-seeded membrane to another media compartment.

SIS scaffolds will be prepared as described in Example 1. Microvascular endothelial cells (mvEC), isolated following conventional methods, will be seeded onto one side of the scaffold. hKDCs will subsequently be seeded onto the other side of the scaffolds. The scaffolds will then be placed into bioreactor chambers, which allow for media flow across each side of the scaffolds, and cultured to allow monolayer formation as in Example 6. Monolayer formation will be monitored by TEER measurement and FITC-inulin leakage as in Example 6. At this point, each media compartment is enclosed in a small loop, which is separated only by the intact cell monolayers on the SIS scaffold. For glucose measurements, media containing known concentrations of glucose will be used in the different media compartments with or without the addition of a glucose transport inhibitor such as e.g. phloridzin. The media will be allowed to flow across the mature cell monolayers for a period of approximately 48 hours, with media samples being periodically removed for glucose concentration measurements via a colorimetric assay. Multiple experiments will be conducted with differing glucose concentrations in the two compartments in order to examine the change in glucose concentrations with time. Results from these measurements will be used to calculate the relative amount of glucose transport vs. consumption by the cells with respect to time. Similar experiments can be conducted with other solutes that are either transported or taken up and degraded by the cells, such as *e.g.* albumin.

### Example 8: Vitamin D activation

Vitamin D activation is a specific function of proximal tubule cells of the kidney. Accordingly, this example tests for vitamin D activation by testing the activity of 25-(OH)D₃-12-hydroxylase enzyme which converts the inactive 25-OH-D₃ precursor into its active 1,25-(OH)₂D₃ form.

To assay the vitamin D activation, hKDC will be seeded on SIS, which will be prepared as described in Example 1. hKDC at passage 4 will be seeded onto the SIS scaffolds at 5x104 cells/scaffold and cultivated for three weeks with REGM™ renal epithelial growth medium (Lonza, Walkersville).

The medium will be exchanged to a medium containing the inactive 25-OH-D₃ precursor. After an incubation time of approximately 15 minutes to 2 hours, the medium will be collected and analyzed for the amount of both precursor and the active 1,25-(OH)₂D₃ form by HPLC analysis or ELSIA. The incubation time may be varied depending on incubation medium and incubation temperature. The conversion can be further induced by the addition of parathyroid hormone or inhibited by phosphate addition.

### Example 9: hKDC/SIS Renal Proximal Tubule System for Nephrotoxicity Testing and Drug Discovery Applications

In this example, the effects of specific nephrotoxic substances (*e.g*. cisplatin, vinblastin) or renoprotective reagents will be applied on the hKDC/SIS renal proximal tubule model system. To do this, hKDCs will be seeded onto SIS, under static and/or flow conditions and allowed to grow and differentiate into a monolayer of proximal tubule epithelium. Then various nephrotoxic substances will be applied to the hKDC/SIS system and cell viability, morphology and proximal tubule functionality will be evaluated. Renal functional parameters include, *e.g.*, solute transport, TEER, inulin leakage, albumin uptake, vitamin D synthesis, erythropoietin and prostaglandin production. In addition, the hKDC proximal tubule system will be tested for its ability to detect the effects of various renoprotective and other cytoprotective reagents.

### Example 10: Isolation of Human Kidney-Derived Cells

Normal human kidneys were obtained from the National Disease Research Interchange (NDRI, Philadelphia, PA). Each kidney was washed in Dulbecco's modified Eagles medium (DMEM-low glucose, Invitrogen, Carlsbad, CA) or phosphate buffered saline (PBS, Invitrogen) in order to remove blood and debris. Tissue was dissected from the outer cortex region, inner medullar region, and subcapsular region of the kidney. The tissues were then mechanically dissociated in tissue culture plates until the tissue was minced to a fine pulp. The tissue was then transferred to a 50-milliliter conical tube. The tissue was then digested in either good manufacturing practice (GMP) enzyme mixtures containing 0.25 units PZ activity/milliliter collagenase (NB6, N0002779, Serva Electrophoresis GmbH, Heidelberg, Germany), 2.5 units/milliliter dispase (Dispase II 165 859, Roche Diagnositics Corporation, Indianapolis, IN), 1 unit/milliliter hyaluronidase (Vitrase, ISTA Pharmaceuticals, Irvine, Ca) or non-GMP grade enzyme mixtures containing 500 units/milliliter collagenase (Sigma, St Louis, MO), 50 units/milliliter dispase (Invitrogen) and 5 units/milliliter hyaluronidase (Sigma). Kidney-derived cells were also isolated with 50 units/milliliter dispase. The enzyme mixture was combined with either renal epithelial growth medium (REGM) (Cambrex, Walkersville, MD) or mesenchymal stem cell growth medium (MSCGM) (Cambrex). The conical tubes containing the tissue, medium and digestion enzymes were incubated at 37°C in an orbital shaker at 225 rpm for 1 hour.

The digest was centrifuged at 150 x g for 5 minutes and the supernatant was aspirated. The resulting cell pellet was resuspended in 20 milliliters of REGM or MSCGM. The cell suspension was filtered through a 40-micron nylon BD FALCON cell strainer (BD Biosciences, San Jose, CA). The filtrate was resuspended in medium (total volume 50 milliliters) and centrifuged at 150 x g for 5 minutes. The supernatant was aspirated and the cell pellet was resuspended in 50 milliliters of fresh culture medium. This process was repeated twice more.

After the final centrifugation, the supernatant was aspirated and the cell pellet was resuspended in 5 milliliters of fresh culture medium. The number of viable cells was determined using a Guava instrument (Guava Technologies, Hayward, CA). Cells were then plated at a seeding density of 5000 cells/cm² onto 2% gelatin or laminin coated tissue culture flasks and cultured either in a low oxygen (hypoxia) or normal (normoxia) atmosphere. Table 1 shows the donor information and growth conditions used to isolate populations of kidney-derived cells. To obtain single-cell derived clones of kidney cells, limiting dilution techniques were performed. In total, cells were isolated using twenty-four different conditions, from four different cadaveric donors ages 39, 46, 21 and 10 years old.

### Example 11: Morphology of Human Kidney-Derived Cells

Seven days after isolation, kidney-derived cell populations were assessed by light microscopy and morphological characteristics of the cells were observed. Consistently, all isolation conditions gave rise to cells with an epithelial morphology. *(see* Table 11-1).

| **Table 11-1:** Conditions used to establish cultures of kidney-derived cells. | | | | | | | | |
|---|---|---|---|---|---|---|---|---|
| **Isolation** | **Age** | **Donor gender** | **Tissue source** | **Enzymes** | **Media** | **Substrate** | **Atm** | **Morphology** |
| 1 | 39 | Male | Cortex | A | REGM | Gelatin | N | Epithelial |
| 2 | 39 | Male | Medulla | A | REGM | Gelatin | N | Epithelial |
| 3 | 39 | Male | Cortex | A | MSCGM | Gelatin | N | Epithelial |
| 4 | 39 | Male | Medulla | A | MSCGM | Gelatin | N | Epithelial |
| | | | | | | | | |
| 5 | 39 | Male | Cortex | A | REGM | Gelatin | H | Epithelial |
| 6 | 39 | Male | Medulla | A | REGM | Gelatin | H | Epithelial |
| 7 | 39 | Male | Cortex | A | MSCGM | Gelatin | H | Epithelial |
| 8 | 39 | Male | Medulla | A | MSCGM | Gelatin | H | Epithelial |
| | | | | | | | | |
| 9 | 39 | Male | Cortex | A | REGM | Laminin | N | Epithelial |
| 10 | 39 | Male | Medulla | A | REGM | Laminin | N | Epithelial |
| 11 | 39 | Male | Cortex | A | MSCGM | Laminin | N | Epithelial |
| 12 | 39 | Male | Medulla | A | MSCGM | Laminin | N | Epithelial |
| | | | | | | | | |
| 13 | 39 | Male | Cortex | A | REGM | Laminin | H | Epithelial |
| 14 | 39 | Male | Medulla | A | REGM | Laminin | H | Epithelial |
| 15 | 39 | Male | Cortex | A | MSCGM | Laminin | H | Epithelial |
| 16 | 39 | Male | Medulla | A | MSCGM | Laminin | H | Epithelial |
| | | | | | | | | |
| 17 | 46 | Male | Subcapsular | B | REGM | Gelatin | N | Epithelial |
| 18 | 46 | Male | Cortex | B | REGM | Gelatin | N | Epithelial |
| 19 | 46 | Male | Cortex | A | REGM | Gelatin | N | Epithelial |
| 20 | 46 | Male | Medulla | B | REGM | Gelatin | N | Epithelial |
| 21 | 46 | Male | Medulla | A | REGM | Gelatin | N | Epithelial |
| | | | | | | | | |
| 22 | 21 | Male | Subcapsular | A | REGM | Gelatin | N | Epithelial |
| 23 | 21 | Male | Cortex | A | REGM | Gelatin | N | Epithelial |
| | | | | | | | | |
| 24 | 10 | Female | Cortex | C | REGM | Gelatin | N | Epithelial |
| Non-GMP grade enzymes (A). GMP-grade enzymes (B). Dispase (C). Age of donor in years (Age). Atmosphere cultures were grown (Atm). Normoxia (N). Hypoxia (H). | | | | | | | | |

This data illustrates that kidney-derived cells can be isolated from a donor of any age or gender, as well as isolated using various growth media formulations or culture conditions. The ease and consistency of the isolation procedure shows that kidney-derived cells are a valuable source of cells for use in cell-based therapies.

### Example 12: Kidney-Derived Cell Growth Potential

Kidney-derived cells can be extensively propagated in culture and are able to generate significant numbers of cells in a short time. This is a criterion for the development of allogeneic cell therapies.

Kidney-derived cells were plated at 5,000 cells/cm² onto T75 flasks in REGM or MSCGM and cultured at 37°C in 5% carbon dioxide. Cells were passaged every 2-5 days. At each passage, cells were counted and viability was measured using a Guava instrument (Guava Technologies, Hayward, CA). Cell populations were continually passaged for several weeks until senescence was reached. Senescence was determined when cells failed to achieve greater than one population doubling during the study time interval. Population doublings [ln(final cell yield/initial number of cells plated)/ln2] were then calculated.

For karyotype analysis, passage 4 and passage 10 kidney-derived cells, from isolations 22 and 23, were plated into T25 flasks and allowed to attach overnight. Flasks were then filled with REGM and karyotype analysis was performed.

Table 12-1 is a summary of the growth data for isolations tested. There was no noticeable effect on the cell growth characteristics with regards to donor age, tissue source, or enzymes used to isolate the cells.

Karyotype analysis was performed on isolations 22 and 23 at both passage 4 and passage 10. Both demonstrated a normal karyotype at passage 4 and passage 10.

| **Table 12-1:** Summary of growth potential data. Population doublings (PD). Refer to Table 11-1 for isolation number cross-reference. | | | | |
|---|---|---|---|---|
| **Isolation** | **Days until senescence** | **Passage** | **PD** | **Viability (%)** |
| 1 | 54 | 12 | 31.2 | 98 |
| 2 | 54 | 12 | 26.8 | 98 |
| 17 | 51 | 11 | 30.2 | 98 |
| 18 | 48 | 10 | 26.8 | 97 |
| 19 | 42 | 9 | 24.9 | 97 |
| 20 | 48 | 10 | 31.0 | 98 |
| 21 | 48 | 10 | 29.0 | 98 |
| 22 | 47 | 16 | 28.7 | 97 |
| 23 | 47 | 16 | 27.9 | 97 |

On average, population doublings (PD) at senescence was 28.5, while the average viability was 97.6%.

In summary, kidney-derived cells have a robust growth potential in culture. These data can be used to estimate the total number of cells generated from one whole human kidney. If all of the kidney tissue was processed, and the resulting cells were cultured for 31 population doublings, one whole human kidney would yield an estimated 1.89 x10¹⁶ total cells. Therefore, considering that one therapeutic dose of cells is 1 x 10⁸ cells per person, kidney-derived cells, isolated from a single kidney will be sufficient to treat 189 million patients. Ultimately, these cells are a highly expandable source of cells for use in allogeneic-based cell therapies.

### Example 13: hKDC Surface Marker Phenotype

Flow cytometric analysis was performed on human kidney-derived cells to determine the surface marker phenotype. Cells from 9 of the isolations in Example 11 were expanded to passage 4 and passage 10 in REGM on T75 flasks at 37°C and 5% carbon dioxide. Adherent cells were washed in PBS and detached with TrypLE Select (Gibco, Grand Island, NY). Cells were harvested, centrifuged and resuspended in 3% (v/v) FBS in PBS at a concentration of 2 x 10⁵ cells/milliliter. The specific antibody was added to 100 microliters of cell suspension and the mixture was incubated in the dark for 30-45 minutes at 4°C. After incubation, cells were washed with PBS and centrifuged to remove excess antibody. Cells were resuspended in 500 microliters PBS and analyzed by flow cytometry. Flow cytometry analysis was performed with a Guava Instrument (Guava Technologies, Hayward, CA). Antibodies used to characterize the surface marker phenotype are shown in Table 13-1.

**Table 13-1: Antibodies used in characterizing cell the surface marker phenotype of kidney-derived cells.**

| **Antibody** | **Manufacture** | **Catalog number** |
|---|---|---|
| CD34 | BD Pharmingen | 555821 |
| CD44 | BD Pharmingen | 555478 |
| CD45R | BD Pharmingen | 555489 |
| CD117 | BD Pharmingen | 340529 |
| CD141 | BD Pharmingen | 559781 |
| CD31 | BD Pharmingen | 555446 |
| CD49c | BD Pharmingen | 556025 |
| CD73 | BD Pharmingen | 550257 |
| CD90 | BD Pharmingen | 555596 |
| HLA-I | BD Pharmingen | 555553 |
| HLA-II | BD Pharmingen | 555558 |
| CD133 | Miltenyi Biotech | 120-001-243 |
| SSEA4 | R&D Systems | FAB1435P |
| CD105 | SantaCruz Biotech | SC-21787 |
| CD104 | BD Pharmingen | 555720 |
| CD166 | BD Pharmingen | 559263 |
| CD29 | BD Pharmingen | 555442 |
| CD24 | BD Pharmingen | 555428 |
| CD56 | AbCAM | MEM188 |
| CD138 | BD Pharmingen | 550805 |
| CD80 | BD Pharmingen | 557226 |
| CD86 | BD Pharmingen | 555659 |
| E-cadherin | BD Pharmingen | 612130 |
| IgG-FITC | BD Pharmingen | 555748 |
| IgG-PE | BD Pharmingen | 555749 |

Table 13-2 shows a summary of all surface marker phenotype data. All isolations tested showed positive staining for CD24, CD29, CD44, CD49c, CD73, CD166, SSEA-4 and HLA I and negative staining for CD31, CD34, CD45, CD56, CD80, CD86, CD104, CD105, CD117, CD133, CD138, CD141, E-cadherin and HLA II. In addition, all isolations analyzed were expanded for multiple generations (passage 10) and still retained their surface marker phenotype.

These cells express HLA I, but do not express HLA II, CD80 or CD86. These cell expression characteristics reflect the cell's ability to evade a host immune system. These data demonstrate that kidney-derived cells are non-immunogenic and can be administered to a patient without the need for tissue typing or immunosuppression.

In summary, these data demonstrate that kidney-derived cells from multiple donors can be isolated under various conditions (*see* Table 11-1) and still maintain their surface marker phenotype. In addition, they express putative progenitor markers such as CD24 and SSEA-4, yet do not express mature, lineage-committed markers such as E-cadherin. Finally, kidney-derived cells are non-immunogenic and therefore are an attractive source of cells for use in allogeneic cell therapies.

**Table 13-2: Summary of surface marker analysis. Not determined (ND). Positive staining (+). Negative staining (-).**

| **Surface markers** | **Alternate name** | Isolation | | | | | | | | | |
|---|---|---|---|---|---|---|---|---|---|---|---|
| | | 1 | 2 | 17 | 18 | 19 | 20 | 21 | 22 | 23 | 24 |
| CD29 | B1-integrin | + | + | + | + | + | + | + | + | + | + |
| CD44 | HCAM | + | + | + | + | + | + | + | + | + | + |
| CD49C | a3-integrin | ND | ND | + | + | + | + | + | + | + | + |
| CD166 | ALCAM | + | + | + | + | + | + | + | + | + | + |
| CD24 | Heat shock antigen-1 | ND | ND | + | + | + | + | + | + | + | + |
| CD73 | SH3 | ND | ND | + | + | + | + | + | + | + | + |
| CD90 | Thy-1 | ND | ND | + | + | + | + | + | + | + | + |
| SSEA4 | none | + | + | + | + | + | + | + | + | + | + |
| CD31 | PECAM-1 | - | - | - | - | - | - | - | - | - | - |
| CD34 | gp105 | - | - | - | - | - | - | - | - | - | - |
| CD45 | Ly5 | - | - | - | - | - | - | - | - | - | - |
| CD56 | NCAM | ND | ND | - | - | - | - | - | - | - | - |
| CD104 | b4-integrin | - | - | - | - | - | - | - | - | - | - |
| CD138 | Syndecan-1 | ND | ND | - | - | - | - | - | - | - | - |
| CD141 | Thrombomodulin | - | - | - | - | - | - | - | - | - | - |
| E-CADHERIN | none | - | - | - | - | - | - | - | - | - | - |
| CD105 | Endoglin | - | - | - | - | - | - | - | - | - | - |
| CD117 | c-Kit | - | - | - | - | - | - | - | - | - | - |
| CD133 | AC133 | - | - | - | - | - | - | - | - | - | - |
| CD80 | B7-1 | ND | ND | ND | ND | ND | ND | ND | - | - | - |
| CD86 | B7-2 | ND | ND | ND | ND | ND | ND | ND | - | - | - |
| HLA I | MHC-a,b,c | ND | ND | ND | ND | ND | ND | ND | + | + | + |
| HLAII | MHC-DP, DQ, DR | ND | ND | ND | ND | ND | ND | ND | - | - | - |

### Example 14: Kidney-Derived Cell Gene Expression

RNA was extracted from cells from isolations 1, 2 and 17-23 using an RNA extraction kit (RNeasy Mini Kit; Qiagen, Valencia, CA). RNA was eluted with 50 microliters DEPC-treated water and stored at -80°C. RNA was reversed transcribed using random hexamers with the TaqMan reverse transcription reagents (Applied Biosystems, Foster City, CA) at 25°C for 10 minutes, 37°C for 60 minutes and 95°C for 10 minutes. Samples were stored at -20°C. Using the primers described in Table 14-1, selected genes were investigated by conventional PCR (polymerase chain reaction). PCR was performed on cDNA samples using RT² PCR primer sets (SuperArray Biosciences Corp, Frederick MD).

| **Table 14-1:** Primers used in the study | |
|---|---|
| **Gene** | **SuperArray catalogue number** |
| Oct-4 | PPH02394A |
| Rex 1 | PPH02395A |
| Sox2 | PPH02471A |
| Human TERT (hTERT) | PPH00995A |
| FGF4 | PPH00356A |
| Pax 2 | PPH06881A |
| Cadherin-11 | PPH00667A |
| WT1 | PPH00254A |
| FOXD1 | PPH01990A |
| WNT4 | PPH02445A |
| Epo | PPH01338A |
| EpoR | PPH02642A |
| Eya1 | PPH10542A |
| HNF3B | PPH00976A |
| Sox17 | PPH02451A |
| Gata4 | PPH010860A |
| Six2 | PPH10860A |
| CXCR4 | PPH00621A |
| BMP-2 | PPH00549A |
| BMP-7 | PPH00527A |
| GDF5 | PPH01912A |

Primers were mixed with 1 microliter of cDNA and 2X ReactionReady™ SYBR Green PCR Master Mix (SuperArray Biosciences) according to manufacturer's instructions and PCR was performed using an ABI Prism 7000 system (Applied Biosystems, Foster City, CA). Thermal cycle conditions were initially 50°C for 2 min and 95°C for 10 min followed by 34 cycles of 95°C for 15 sec and 60°C for 1 min. For GAPDH, PCR was performed using GAPDH primers from Applied Biosystems (cat#: 402869) 1 microliter of cDNA solution and 1× AmpliTaq Gold universal mix PCR reaction buffer (Applied Biosystems, Foster City, CA) according to manufacturer's instructions. Primer concentration in the final PCR reaction was 0.5 micromolar for both the forward and reverse primer and the TaqMan probe was not added. Samples were run on 2% (w/v) agarose gel and stained with ethidium bromide (Sigma, St. Louis, MO). Images were captured using a 667 Universal Twinpack film (VWR International, South Plainfield, NJ) and a focal-length Polaroid™ camera (VWR International, South Plainfield, NJ). For each gene analyzed, the final PCR product was excised from the gel and target sequence was confirmed by DNA sequencing.

### RT-PCR Analysis

All cell isolates analyzed showed a constant and stable gene expression profile. RT-PCR analysis was performed on isolations 1, 2, and 17-23 in order to detect the expression of early developmental gene marker (Oct-4, Rex-1, Sox2, FGF4, hTert), kidney developmental gene markers (Pax-2, WT-1, Eya-1, Wnt-4, BMP-7, Cadherin-11, FoxD1), metanephric mesenchymal gene markers (Pax-2, Eya-1, WT-1, Six2, and FoxD1), and genes that promote the survival of metanephric mesenchyme (BMP-7). In addition, the expression of other developmental genes, such as endodermal genes (HNF3B, CXC-R4, Sox-17, GATA-4) as well as gene markers that promote renal repair or have therapeutic value in treating kidney disease (Epo, EpoR, BMP-7, BMP-2, GDF5) were analyzed.

As shown in Table 14-2, all isolations showed positive expression for Oct-4 and Rex-1 and negative expression for Sox2, FGF4, hTERT and Wnt-4. Isolations 17-23 showed positive expression for Pax-2, WT1, Cadherin-11 and FoxD1. Isolations 22 and 23 showed positive expression for Eya-1, Sox-17 and CXCR-4 and negative expression for GATA-4. Isolations 17-23 expressed EpoR, but did not express Epo. Isolations 17-22 expressed BMP-2, BMP-7 and GDF5. In addition, all isolations can be expanded multiple generations (passage 10) and still retain their gene expression phenotype.

**Table 14-2: Summary of gene expression analysis. Positive expression (+). Negative expression (-). Not determined (ND). Genes that function during early development (Early development). Genes that function during kidney development (Kidney development). Metanephric mesenchymal markers (Met). Endodermal lineage markers (Endoderm). Genes involved in kidney survival (renoprotective). Genes involved in metanephric mesenchyme survival (Survival).**

| | | **Isolation** | | | | | | | | |
|---|---|---|---|---|---|---|---|---|---|---|
| **Gene name** | **Function** | 1 | 2 | 17 | 18 | 19 | 20 | 21 | 22 | 23 |
| Oct4/pfu | Early development | + | + | + | + | + | + | + | + | + |
| Rex-1 | Early development | + | + | + | + | + | + | + | + | + |
| Sox2 | Early development | - | - | - | - | - | - | - | - | - |
| FGF4 | Early development | - | - | - | - | - | - | - | - | - |
| hTert | Early development | - | - | - | - | - | - | - | - | - |
| Pax2 | Kidney development, Met | ND | ND | + | + | + | + | + | + | + |
| Cadherin-11 | Kidney development | ND | ND | + | + | + | + | + | + | + |
| FoxDl | Kidney development, Met | ND | ND | + | + | + | + | + | + | + |
| WT-1 | Kidney development, Met | ND | ND | + | + | + | + | + | + | + |
| Eya1 | Kidney development | ND | ND | ND | ND | ND | ND | ND | + | + |
| Wnt-4 | Kidney development | ND | ND | ND | ND | ND | ND | ND | - | - |
| SIX2 | Met | ND | ND | ND | ND | ND | ND | ND | - | - |
| GATA-4 | Endoderm | ND | ND | ND | ND | ND | ND | ND | - | - |
| HNF3B | Endoderm | ND | ND | ND | ND | ND | ND | ND | + | + |
| CXC-R4 | Endoderm | ND | ND | ND | ND | ND | ND | ND | + | + |
| Sox-17 | Endoderm | ND | ND | ND | ND | ND | ND | ND | + | + |
| Epo | Renoprotective | ND | ND | - | - | - | - | - | - | - |
| EpoR | Renoprotective | ND | ND | + | + | + | + | + | + | + |
| BMP2 | Renoprotective | ND | ND | + | + | + | + | + | ND | ND |
| GDF5 | Renoprotective | ND | ND | + | + | + | + | + | ND | ND |
| BMP7 | Kidney development, Survival | ND | ND | + | + | + | + | + | ND | ND |

In summary, kidney-derived cells express genes involved in early development and kidney development. They express markers for metanephric mesenchyme and markers for renal progenitor cells. They express endodermal markers as well as factors involved in renal repair and tubulogenesis.

In total, these data demonstrate that kidney-derived cells are a source of putative renal progenitor cells that can be used for cell-based therapies to protect or repair damaged kidney tissue.

### Example 15: Trophic Factor Secretion Analysis

Kidney-derived cells were shown to consistently produce trophic factors that protect and repair the kidney. Therefore, these cells may serve as a therapeutic agent for treating kidney disease.

Passage 3 cells, from isolations 17-21 were seeded at 5,000 cells/cm² in one T75 flask/isolation, each containing 15 milliliters of REGM. Cells were cultured at 37°C in 5% carbon dioxide. After overnight culture, the medium was changed to a serum-free medium (DMEM-low glucose (Gibco), 0.1% (w/v) bovine serum albumin (Sigma), penicillin (50 units/milliliter) and streptomycin (50 micrograms/milliliter) (Gibco)) and further cultured for 8 hours. Conditioned, serum-free medium was collected at the end of incubation by centrifugation at 14,000 x g for 5 min and stored at -20°C.

Cells were washed with PBS, detached using 4 milliliters TrypLE Select (Gibco) and counted with a Guava instrument (Guava Technologies, Hayward, CA) to estimate the number of cells in each flask. Using Searchlight Proteome Arrays (Pierce Biotechnology Inc), samples were then assayed by ELISA for the following trophic factors: tissue inhibitor of metalloproteinase-1 (TIMP-1), tissue inhibitor of metalloproteinase-2 (TIMP-2), platelet-derived epithelial growth factor bb (PDGF-bb), keratinocyte growth factor (KGF), hepatocyte growth factor (HGF), basic fibroblast growth factor (FGF2), vascular endothelial growth factor (VEGF), Heparin-binding epidermal growth factor (HB-EGF), monocyte chemotactic protein-1 (MCP-1), interleukin-6 (IL-6), interleukin-8 (IL-8), transforming growth factor alpha (TGFα), brain-derived neurotrophic factor (BDNF), stromal-derived factor 1b (SDF1b), cilliary neurotrophic factor (CNTF), basic nerve growth factor (b-NGF), neurotrophin-3 (NT-3), growth-related oncogene-alpha (GRO-α), interleukin-lb (IL-1b), interleukin-12p40 (IL-12p40), interleukin-12p70 (IL-12p70), interleukin-11 (IL-11), interleukin-15 (IL-15), matrix metalloproteinase-2 (MMP-2), matrix metalloproteinase-9 (MMP-9), angiopoietin-2 (ANG-2) and human growth hormone (HGH).

### Analysis of Trophic Factor Production

The secretion of twenty-seven different growth factors and cytokines were analyzed on isolations 17-21. The results are summarized in Table 15-1. All isolations secreted TIMP-1, TIMP-2, VEGF, and MMP-2 at over 300 picograms/milliliter/1x10⁶ cells/8 hours. They secreted 50-300 picograms/milliliter/1x10⁶ cells/8 hours of FGF2 and HGF and 1-50 picograms/milliliter/1x10⁶ cells/8 hours of KGF, PDGF-bb, b-NGF, IL-12p40 and IL-11. SDF-1, ANG-2, HGH and Il-12p70 were not detected.

In summary, this data shows that kidney-derived cells secrete several trophic factors for protecting and repairing damaged kidney tissue. For example, FGF2, HGF, and TGFα have been implicated in renal repair. Kidney-derived cells secrete these factors at elevated and consistent levels. Therefore, these cells are a valuable source of cells for use in therapies targeting kidney diseases.

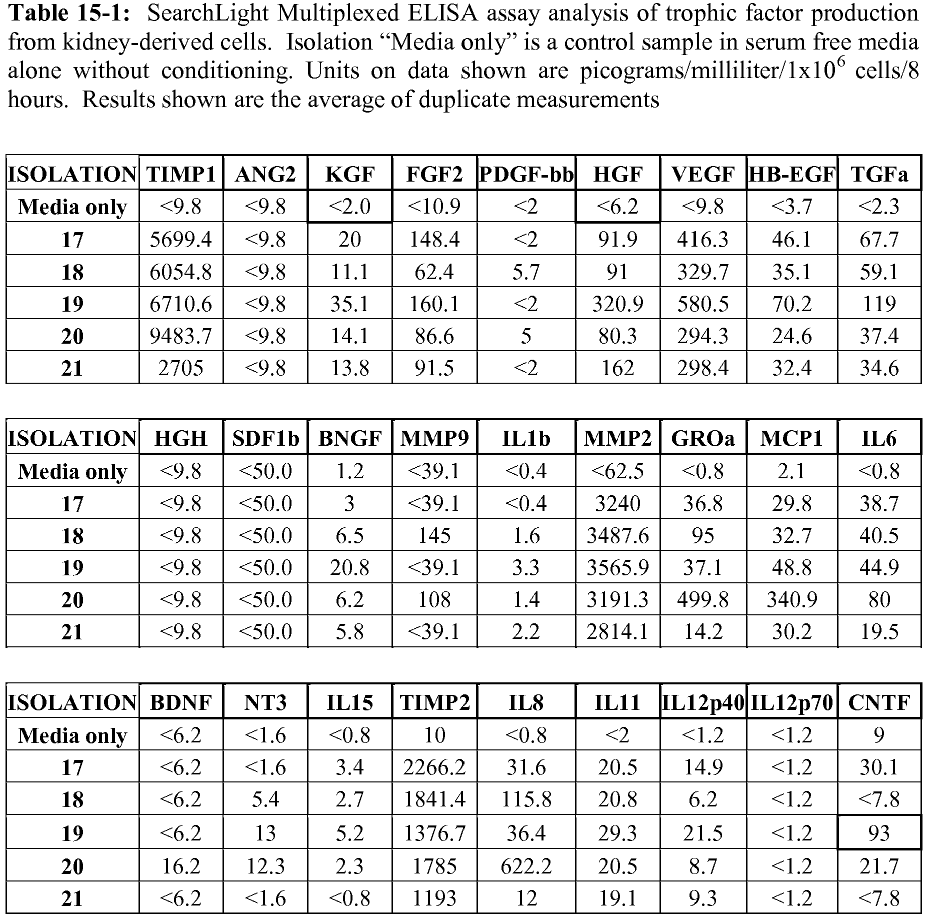

### Example 16: Kidney-Derived Cell Tubulogenesis In Vitro

Kidney-derived cells can be thawed at passage 4 and passage 10 and then triturated into a single-cell suspension at 4 x 10⁴ cells/milliliter in a type I collagen solution containing 66% vitrogen 100 (3 milligrams/milliliter (Cohesion Technologies, Palo Alto, CA). Cells in suspension can be plated onto a de-cellularized omentum membrane. The collagen/cell mixture can then be incubated for 30 minutes at 37°C, 5% CO₂, 95% air to allow the collagen to gel and then culture medium is added. Cells are fed every 3 days for 7 days. On day 7, cultures are treated with varying concentrations of hepatocyte growth factor and further cultured until tubulogenesis is observed.

These observations demonstrate that kidney-derived cells can self-organize into tubule structures *in vitro.* These structures have value as building blocks for kidney reconstruction applications as well as for developing drug screening and toxicology assays.

### Example 17: Kidney-Derived Cell Tubulogenesis in vivo

Three 35/65 PCL/PGA (10 cm diameter x 2 mm thickness) films were seeded with human kidney-derived cells (10,000cells/cm²) and cultured in REGM (Cambrex) at 37°C and 5% carbon dioxide for 8 days. The 35/65 PCL/PGA foam scaffold was prepared according to the methods described in U.S. patent 6,355,699, incorporated herein by reference in its entirety. The cell/film constructs were then removed from the film-casting dish, stacked into three layers and applied to a 35/65 PCL/PGA foam scaffold support. This construct was then cultured for an additional 24 hours and then cut into 3 x 3 mm square pieces prior to implantation. The implants were then washed with PBS and transferred to a 6-well plate filled with PBS for transport.

The implants were subcutaneously implanted bilaterally in the dorsal lateral thoracic-lumbar region of SCID mice. Male SCID mice (Fox Chase SCID CB17SC strain) were purchased from Taconic Inc., (Hudson, NY). and were 5 weeks old. Two implants were placed in each SCID mouse. Two skin incisions, each approximately 5 mm in length, were made on the dorsum of the mice. The incisions were placed transversely over the lumbar area about 5 mm cranial to the palpated iliac crest, with one on either side of the midline. The skin was then separated from the underlying connective tissue to make a small pocket, and the implant was placed about 10 mm cranial to the incision. The skin incisions were closed with Reflex 7 metal wound clips. After 3 weeks, the implants were removed from the subcutaneous pocket, fixed in 10% formalin, embedded in paraffin wax, sectioned, stained with hematoxylin and eosin (H&E) and evaluated by a pathologist using light microscopy techniques.

Kidney-derived cells formed tubule-like structures within the layers of PCL/ PGA film. These tubules showed a distinct epithelial wall and a clear lumen. Kidney-derived cells infiltrated the foam scaffold, deposited extracellular matrix, and formed a dense, tissue-like structure. In addition, kidney-derived cells within the foam stimulated angiogenesis and the formation of vascular networks.

In summary, human kidney-specific cells formed tubule structures after exposure to an *in vivo* microenvironment. The ability of kidney-derived cells to respond to microenvironmental signals and to instruct the cells to form tubules, further illustrates the renal progenitor nature of these cells. In addition, the cells migrated into the foam scaffolds, forming a tissue-like structure that promoted angiogenesis. This data illustrates the utility of kidney-derived cells as cellular building blocks for reconstructing kidney tubules and ultimately for use in kidney tissue engineering applications.

While the invention has been described and illustrated herein by references to various specific materials, procedures and examples, it is understood that the invention is not restricted to the particular combinations of material and procedures selected for that purpose. Numerous variations of such details can be implied as will be appreciated by those skilled in the art. It is intended that the specification and examples be considered as exemplary, only, with the true scope of the invention being indicated by the following claims.

## Claims

1. A bioartificial proximal tubule device comprising a decellularized biological matrix scaffold derived from mammalian tissue and seeded with one or more precursor cells under conditions sufficient to allow the differentiation of the precursor cell into renal proximal tubule epithelial cells, wherein the differentiated cells form an epithelial monolayer on the scaffold, and wherein the one or more precursor cells is selected from the group consisting of primary renal tubule epithelial cells, inducible pluripotent stem cells, renal progenitor cells, progenitor cells differentiated into renal cells or renal progenitor cells, stem cells isolated from the kidney or progenitor cells isolated from the kidney, and mixtures thereof; wherein the decellularized biological matrix scaffold is derived from mucosal or submucosal tissue, or wherein
the decellularized biological matrix scaffold is derived from a mammalian alimentary canal.

2. A bioartificial proximal tubule device comprising a decellularized biological scaffold derived from mammalian tissue and having at least two surfaces wherein at least one surface is seeded with one or more precursor cells under conditions sufficient to allow differentiation of the cells into renal proximal tubule epithelial cells, wherein the cells form an epithelial monolayer on the surface of the scaffold, and wherein the one or more precursor cells is selected from the group consisting of primary renal tubule epithelial cells, inducible pluripotent stem cells, renal progenitor cells, progenitor cells differentiated into renal cells or renal progenitor cells, stem cells isolated from the kidney or progenitor cells isolated from the kidney, and mixtures thereof; wherein
the decellularized biological matrix scaffold is derived from mucosal or submucosal tissue, or wherein
the decellularized biological matrix scaffold is derived from a mammalian alimentary canal.

3. A method of differentiating one or more precursor cells into renal cells comprising seeding a decellularized biological matrix scaffold derived from mammalian tissue with one or more precursor cells and culturing the cells on the scaffold under conditions sufficient to allow the differentiation of the precursor cell into renal proximal tubule epithelial cells, wherein the differentiated cells form an epithelial monolayer on the scaffold.

4. The method of claim 3, wherein the decellularized biological matrix scaffold is derived from mucosal or submucosal tissue.

5. The method of claim 3, wherein the decellularized biological matrix scaffold is derived from a mammalian alimentary canal.

6. The bioartificial proximal tubule device of claims 1 or 2 or the method of claim 5, wherein the decellularized biological matrix scaffold derived from mammalian alimentary canal is derived from the stomach, duodenum, jejunum, ileum or colon of a mammal.

7. The method of claim 3, wherein the one or more precursor cells is selected from the group consisting of primary renal tubule epithelial cells, inducible pluripotent stem cells differentiated into renal cells or renal progenitor cells, progenitor cells differentiated into renal cells or renal progenitor cells, stem cells isolated from the kidney or progenitor cells isolated from the kidney, and mixtures thereof.

8. The bioartificial proximal tubule device of claims 1, 2, or 6 or the method of claims 3 to 7, wherein the progenitor cells isolated from the kidney are human kidney-derived cells.

9. The bioartificial proximal tubule device of claim 8 or the method of claim 8, wherein the human kidney-derived cells are capable of self-renewal and expansion in culture and are positive for expression of at least one of Oct-4, Rex-1, Pax-2, Cadherin-11, FoxD1, WT1, Eya1, HNF3B, CXC-R4, Sox-17, EpoR, BMP2, BMP7, or GDF5; and negative for the expression of at least one of Sox2, FGF4, hTert, Wnt-4, SIX2, E-cadherin or GATA-4.

10. The bioartificial proximal tubule device of claims 8 or 9 or the method of claims 8 or 9, wherein the human kidney-derived cells are positive for at least one of cell-surface markers HLA-I, CD24, CD29, CD44, CD49c, CD73, CD90, CD166, or SSEA-4; and negative for at least one of cell-surface markers HLA II, CD31, CD34, CD45, CD56, CD80, CD86, CD104, CD105, CD117, CD133, CD138, and CD141.

11. The bioartificial proximal tubule device of any of claims 8 to 10 or the method of any one of claims 8 to 10, wherein the human kidney-derived cells secrete at least one of trophic factors FGF2, HGF, TGFα, TIMP-1, TIMP-2, MMP-2 or VEGF; and do not secrete at least one of trophic factors PDGF-bb or IL12p70.

12. The bioartificial proximal tubule device of claim 9 or the method of claim 9, wherein the human kidney-derived cells are capable of self-renewal and expansion in culture and further have one of the following characteristics:
a) positive for the expression of one or more of Oct-4 and Rex-1 and negative for the expression of one or more of Sox2, FGF4, hTert and Wnt-4; or
b) positive for expression of at least one of Eya1, WT1, FoxD1, BMP7, BMP2, GDF5, EpoR or Rex-1, and negative for expression of at least one of Sox2, FGF4, hTert or Wnt-4.

13. The method of claims 3 and 7, wherein the method promotes functioning of the natural regulative mechanisms of contact inhibition and the formation of an intact monolayer without the use of contact inhibitors.

## Patentansprüche

1. Bioartifizielle proximale Tubulusvorrichtung, umfassend ein dezellularisiertes biologisches Matrixgerüst, das aus einem Säugetiergewebe gewonnen wurde und mit einer oder mehreren Präkursorzellen unter Bedingungen beimpft ist, die dazu ausreichen, die Differenzierung der Präkursorzelle in proximale Nierentubulusepithelzellen zu ermöglichen, wobei die differenzierten Zellen eine Epithelmonoschicht an dem Gerüst bilden, und wobei die eine oder die mehreren Präkursorzellen aus der Gruppe bestehend aus primären Nierentubulusepithelzellen, induzierbaren pluripotenten Stammzellen, Nierenprogenitorzellen, in Nierenzellen oder Nierenprogenitorzellen differenzierten Progenitorzellen, aus der Niere isolierten Stammzellen oder aus der Niere isolierten Progenitorzellen und Gemischen davon ausgewählt sind, wobei das dezellularisierte biologische Matrixgerüst aus dem mukosalen oder submukosalen Gewebe gewonnen wird, oder wobei
das dezellularisierte biologische Matrixgerüst aus einem Säugetierverdauungskanal gewonnen wird.

2. Bioartifizielle proximale Tubulusvorrichtung, umfassend ein dezellularisiertes biologisches Gerüst, das aus einem Säugetiergewebe gewonnen wurde und mindestens zwei Oberflächen aufweist, wobei mindestens eine Oberfläche mit einer oder mehreren Präkursorzellen unter Bedingungen beimpft ist, die dazu ausreichen, eine Differenzierung der Zellen in proximale Nierentubulusepithelzellen zu ermöglichen, wobei die Zellen eine Epithelmonoschicht auf der Oberfläche des Gerüsts bilden, und wobei die eine oder die mehreren Präkursorzellen aus der Gruppe bestehend aus primären Nierentubulusepithelzellen, induzierbaren pluripotenten Stammzellen, Nierenprogenitorzellen, in Nierenzellen oder Nierenprogenitorzellen differenzierten Progenitorzellen, aus der Niere isolierten Stammzellen oder aus der Niere isolierten Progenitorzellen und Gemischen davon ausgewählt sind; wobei
das dezellularisierte biologische Matrixgerüst aus einem mukosalen oder submukosalen Gewebe gewonnen wird,
oder wobei
das dezellularisierte biologische Matrixgerüst aus einem Säugetierverdauungskanal gewonnen wird.

3. Verfahren zum Differenzieren von einer oder mehreren Präkursorzellen in Nierenzellen, das das Beimpfen eines dezellularisierten biologischen Matrixgerüstes, das aus Säugetiergewebe mit einer oder mehreren Präkursorzellen gewonnen wurde, und das Kultivieren der Zellen an dem Gerüst unter Bedingungen umfasst, die dazu ausreichen, die Differenzierung der Präkursorzelle in proximale Nierentubulusepithelzellen zu ermöglichen, wobei die differenzierten Zellen eine Epithelmonoschicht an dem Gerüst bilden.

4. Verfahren nach Anspruch 3, wobei das dezellularisierte biologische Matrixgerüst aus einem mukosalen oder submukosalen Gewebe gewonnen wird.

5. Verfahren nach Anspruch 3, wobei das dezellularisierte biologische Matrixgerüst aus einem Säugetierverdauungskanal gewonnen wird.

6. Bioartifizielle proximale Tubulusvorrichtung nach Anspruch 1 oder 2 oder Verfahren nach Anspruch 5, wobei das dezellularisierte biologische Matrixgerüst, das aus einem Säugetierverdauungskanal gewonnen wurde, aus dem Magen, Zwölffingerdarm, Leerdarm, Dünndarm oder Dickdarm des Säugetiers gewonnen wird.

7. Verfahren nach Anspruch 3, wobei die eine oder die mehreren Präkursorzellen aus der Gruppe bestehend aus primären Nierentubulusepithelzellen, induzierbaren pluripotenten Stammzellen, Nierenprogenitorzellen, in Nierenzellen oder Nierenprogenitorzellen differenzierten Progenitorzellen, aus der Niere isolierten Stammzellen oder aus der Niere isolierten Progenitorzellen und Gemischen davon ausgewählt sind.

8. Bioartifizielle proximale Tubulusvorrichtung nach den Ansprüchen 1, 2 oder 6 oder Verfahren nach Anspruch 3-7, wobei die aus der Niere isolierten Progenitorzellen aus der menschlichen Niere gewonnene Zellen sind.

9. Bioartifizielle proximale Tubulusvorrichtung nach Anspruch 8 oder Verfahren nach Anspruch 8, wobei die aus der menschlichen Niere gewonnenen Zellen zur Selbsterneuerung und Expansion in der Kultur in der Lage sind und bei der Expression von mindestens einem von Oct-4, Rex-1, Pax-2, Cadherin-11, FoxD1, WT1, Eyal, HNF3B, CXC-R4, Sox-17, EpoR, BMP2, BMP7 oder GDF5 positiv sind; und bei der Expression von mindestens einem von Sox2, FGF4, hTert, Wnt-4, SIX2, E-Cadherin oder GATA-4 negativ sind.

10. Bioartifizielle proximale Tubulusvorrichtung nach Anspruch 8 oder 9 oder Verfahren nach Anspruch 8 oder 9, wobei die aus der menschlichen Niere gewonnenen Zellen für mindestens einen der Zelloberflächenmarker HLA-I, CD24, CD29, CD44, CD49c, CD73, CD90, CD166 oder SSEA-4 positiv sind; und für mindestens einen der Zelloberflächenmarker HLA II, CD31, CD34, CD45, CD56, CD80, CD86, CD104, CD105, CD117, CD133, CD138 und CD141 negativ.

11. Bioartifizielle proximale Tubulusvorrichtung nach einem der Ansprüche 8 bis 10 oder Verfahren nach einem der Ansprüche 8 bis 10, wobei die aus der menschlichen Niere gewonnenen Zellen mindestens einen der trophischen Faktoren FGF2, HGF, TGFα, TIMP-1, TIMP-2, MMP-2 oder VEGF sekretieren; und mindestens einen der trophischen Faktoren PDGF-bb oder IL12p70 nicht sekretieren.

12. Bioartifizielle proximale Tubulusvorrichtung nach Anspruch 9 oder Verfahren nach Anspruch 9, wobei die aus der menschlichen Niere gewonnenen Zellen zur Selbsterneuerung und Expansion in der Kultur in der Lage sind und ferner eine der folgenden Eigenschaften aufweisen:
a) bei der Expression von einem oder mehreren von Oct-4 und Rex-1 positiv und bei der Expression von einem oder mehreren von Sox2, FGF4, hTert und Wnt-4 negativ; oder
b) bei der Expression von mindestens einem oder mehreren von Eyal, WT1, FoxD1, BMP7, BMP2, GDF5, EpoR oder Rex-1 positiv und bei der Expression von mindestens einem oder mehreren von Sox2, FGF4, hTert oder Wnt-4 negativ.

13. Verfahren nach den Ansprüchen 3 und 7, wobei das Verfahren das Funktionieren der natürlichen regulativen Mechanismen von Kontaktinhibition und Bildung einer intakten Monoschicht ohne Verwendung von Kontaktinhibitoren fördert.

## Revendications

1. Dispositif de tubule proximal bioartificiel comprenant un échafaudage matriciel biologique décellularisé issu de tissu de mammifère et ensemencé avec une ou plusieurs cellules précurseurs dans des conditions suffisantes pour permettre la différenciation de la cellule précurseur en cellules épithéliales de tubule proximal rénal, les cellules différenciées formant une monocouche épithéliale sur l'échafaudage et la ou les cellules précurseurs étant choisies dans le groupe constitué par des cellules épithéliales de tubule rénal primaires, des cellules souches pluripotentes inductibles, des cellules progénitrices rénales, des cellules progénitrices différenciées en cellules rénales ou en cellules progénitrices rénales, des cellules souches isolées à partir du rein ou des cellules progénitrices isolées à partir du rein et des mélanges de celles-ci ; dans lequel
l'échafaudage matriciel biologique décellularisé est issu de tissu muqueux ou sous-muqueux ou dans lequel l'échafaudage matriciel biologique décellularisé est issu d'un canal alimentaire de mammifère.

2. Dispositif de tubule proximal bioartificiel comprenant un échafaudage biologique décellularisé issu de tissu de mammifère et ayant au moins deux surfaces, au moins une surface étant ensemencée avec une ou plusieurs cellules précurseurs dans des conditions suffisantes pour permettre la différenciation des cellules en cellules épithéliales de tubule proximal rénal, les cellules formant une monocouche épithéliale sur la surface de l'échafaudage et la ou les cellules précurseurs étant choisies dans le groupe constitué par des cellules épithéliales de tubule rénal primaires, des cellules souches pluripotentes inductibles, des cellules progénitrices rénales, des cellules progénitrices différenciées en cellules rénales ou en cellules progénitrices rénales, des cellules souches isolées à partir du rein ou des cellules progénitrices isolées à partir du rein et des mélanges de celles-ci ; dans lequel l'échafaudage matriciel biologique décellularisé est issu de tissu muqueux ou sous-muqueux ou dans lequel l'échafaudage matriciel biologique décellularisé est issu d'un canal alimentaire de mammifère.

3. Procédé de différenciation d'une ou plusieurs cellules précurseurs en cellules rénales comprenant l'ensemencement d'un échafaudage matriciel biologique décellularisé issu de tissu de mammifère avec une ou plusieurs cellules précurseurs et la culture des cellules sur l'échafaudage dans des conditions suffisantes pour permettre la différenciation de la cellule précurseur en cellules épithéliales de tubule proximal rénal, dans lequel les cellules différenciées forment une monocouche épithéliale sur l'échafaudage.

4. Procédé selon la revendication 3, dans lequel l'échafaudage matriciel biologique décellularisé est issu de tissu muqueux ou sous-muqueux.

5. Procédé selon la revendication 3, dans lequel l'échafaudage matriciel biologique décellularisé est issu d'un canal alimentaire de mammifère.

6. Dispositif de tubule proximal bioartificiel selon les revendications 1 ou 2 ou procédé selon la revendication 5, dans lesquels l'échafaudage matriciel biologique décellularisé issu de canal alimentaire de mammifère est issu de l'estomac, du duodénum, du jéjunum, de l'iléon ou du côlon d'un mammifère.

7. Procédé selon la revendication 3, dans lequel la ou les cellules précurseurs sont choisies dans le groupe constitué par des cellules épithéliales de tubule rénal primaires, des cellules souches pluripotentes inductibles différenciées en cellules rénales ou en cellules progénitrices rénales, des cellules progénitrices différenciées en cellules rénales ou en cellules progénitrices rénales, des cellules souches isolées à partir du rein ou des cellules progénitrices isolées à partir du rein et des mélanges de celles-ci.

8. Dispositif de tubule proximal bioartificiel selon les revendications 1, 2 ou 6 ou procédé selon les revendications 3 à 7, dans lesquels les cellules progénitrices isolées à partir du rein sont des cellules issues de rein humain.

9. Dispositif de tubule proximal bioartificiel selon la revendication 8 ou procédé selon la revendication 8, dans lesquels les cellules issues de rein humain sont capables d'auto-renouvellement et d'expansion en culture et sont positives pour l'expression de Oct-4, Rex-1, Pax-2, cadhérine-11, FoxD1, WT1, Eya1, HNF3B, CXC-R4, Sox-17, EpoR, BMP2, BMP7 et/ou GDF5 ; et négatives pour l'expression de Sox2, FGF4, hTert, Wnt-4, SIX2, E-cadhérine et/ou GATA-4.

10. Dispositif de tubule proximal bioartificiel selon les revendications 8 ou 9 ou procédé selon les revendications 8 ou 9, dans lesquels les cellules issues de rein humain sont positives pour au moins l'un des marqueurs de surface cellulaire HLA-I, CD24, CD29, CD44, CD49c, CD73, CD90, CD166 ou SSEA-4 ; et négatives pour au moins l'un des marqueurs de surface cellulaire HLA II, CD31, CD34, CD45, CD56, CD80, CD86, CD104, CD105, CD117, CD133, CD138 et CD141.

11. Dispositif de tubule proximal bioartificiel selon l'une quelconque des revendications 8 à 10 ou procédé selon l'une quelconque des revendications 8 à 10, dans lesquels les cellules issues de rein humain sécrètent au moins l'un des facteurs trophiques FGF2, HGF, TGFα, TIMP-1, TIMP-2, MMP-2 ou VEGF ; et ne sécrètent pas au moins l'un des facteurs trophiques PDGF-bb ou IL12p70.

12. Dispositif de tubule proximal bioartificiel selon la revendication 9 ou procédé selon la revendication 9, dans lesquels les cellules issues de rein humain sont capables d'auto-renouvellement et d'expansion en culture et ont en outre l'une des caractéristiques suivantes :
a) elles sont positives pour l'expression de Oct-4 et/ou Rex-1 et négatives pour l'expression de Sox2, FGF4, hTert et/ou Wnt-4 ; ou
b) elles sont positives pour l'expression de Eya1, WT1, FoxD1, BMP7, BMP2, GDF5, EpoR et/ou Rex-1 et négatives pour l'expression de Sox2, FGF4, hTert et/ou Wnt-4.

13. Procédé selon les revendications 3 et 7, le procédé favorisant le fonctionnement des mécanismes régulateurs naturels d'inhibition de contact et la formation d'une monocouche intacte sans l'utilisation d'inhibiteurs de contact.
